# EUROPEAN PATENT SPECIFICATION

(11) **EP 1 161 227 B1**
(45) Date of publication and mention of the grant of the patent: **11.05.2011**
(21) Application number: 00919403.6
(22) Date of filing: 10.03.2000
(51) Int. Cl.: A61K 9/16, A61K 48/00, C12N 15/88

(54) **MICROPARTICLES FOR DELIVERY OF NUCLEIC ACID**
MIKROPARTIKEL ZUR VERABREICHUNG VON NUKLEINSÄUREN
MICROPARTICULES POUR L'ADMINISTRATION DE L'ACIDE NUCLEIQUE

(30) Priority: 11.03.1999 US 266463; 27.05.1999 US 321346
(43) Date of publication of application: 12.12.2001
(73) Proprietor: Eisai Inc., Woodcliff Lake, NJ 07677 (US)
(72) Inventor: LUNSFORD, Lynn, B., Bedford, MA 01730 (US); PUTNAM, David, Cambridge, MA 02141 (US); HEDLEY, Mary, Lynne, Lexington, MA 02173 (US)
(74) Representative: Arends, William Gerrit
(86) International application number: PCT/US2000/006578
(87) International publication number: WO 2000/053161

(56) References cited:
- EP-A- 0 761 211
- WO-A-00/03660
- WO-A-98/31346
- WO-A-98/31398
- WO-A-98/51279
- FR-A- 2 660 556
- GHADERI, RAOUF ET AL: "Effect of preparative parameters on the characteristics of poly (DL- lactide -co- glycolide ) microspheres made by the double emulsion method" INT. J. PHARM. (1996), 141(1,2), 205-216 , XP000946093
- ALONSO MARIA J ET AL: "Determinants of release rate of tetanus vaccine from polyester microspheres." PHARMACEUTICAL RESEARCH (NEW YORK), vol. 10, no. 7, 1993, pages 945-953, XP000946086 ISSN: 0724-8741

## Description

This invention relates to a microparticle for use in a method of therapeutic treatment comprising administering a nucleic acid molecule to an animal.

Gene therapy is a highly promising technique for treatment of hereditary diseases, e.g., cystic fibrosis. Gene therapy can also be used when expression of gene products from genes which are not naturally found in the host cells is desired, for example, from genes encoding cells cytotoxic proteins targeted for expression in cancer. Gene therapy can fall into several categories. It is sometimes desirable to replace a defective gene for the entire lifespan of a mammal, as in the case of an inherited disease such as cystic fibrosis, phenylketonuria, or severe combined immunodeficiency disease (SCID). In other cases, one may wish to treat a mammal with a gene that will express a therapeutic polypeptide for a limited amount of time, e.g., during an infection. Nucleic acids in the form of antisense olignonucleotides or ribozymes are also used therapeutically. Moreover, polypeptides encoded by nucleic acids can be effective stimulators of the immune response in mammals.

Various techniques have been used for introducing genes into cells, including infection with viral vectors, biolistic transfer, injection of "naked" DNA (US patent no. 5580959), and delivery via liposomes or polymeric particles.

### Summary of the Invention

The invention is based on the discovery that microparticles (also called microspheres) containing nucleic acids having an appropriate size for phagocytosis can be made without adversely affecting nucleic acid integrity. These microparticles are highly effective vehicles for the delivery or polynucleotides into phagocytic cells.

In general, the invention features a microparticle for use in a method of therapeutic treatment comprising administering a nucleic acid molecule to an animal by injection into the animal, the microparticle being less than 20 microns in diameter and comprising: a polymeric matrix, a phospholipid; a carbohydrate and a nucleic acid molecule wherein the microparticle is not encapsulated in a liposome and does not comprise a cell. The polymeric matrix includes one or more synthetic polymers having a solubility in water of less than 1 mg/l; in the present context, synthetic is defined as non-naturally occurring. The nucleic acid is either RNA, at least 50% (and preferably at least 70% or even 80%) of which is in the form of closed circles, or circular DNA plasmid molecules, at least 25% (and preferably at least 35%, 40%, 50%. 60%, 70%, or even 80%) of which are supercoiled. The plasmid can be linear or circular. When circular and double-stranded, it can be nicked, i.e., in an open circle, or super-coiled. The nucleic acid, either single-stranded or double-stranded, can also be in a linear form.

The polymeric matrix is made from one or more synthetic polymers having a solubility in water of less than about 1 mg/l. At least 50% (and preferably at least 70% or even 80%) of the nucleic acid molecules are in the form of supercoiled DNA.

The polymeric matrix can be biodegradable. Biodegradable is used herein to mean that the polymers degrade over time into compounds which are known to be cleared from the host cells by normal metabolic pathways. Generally, a biodegradable polymer will be substantially metabolized within about 1 month after injection into a patient, and certainly within about 2 years. In certain cases, the polymeric matrix can be made of a single synthetic, biodegradable copolymer, e.g., poly-lactic-coglycolic acid (PLGA). The ratio of lactic acid to glycolic acid in the copolymer can be within the range of about 1:2 to about 4:1 by weight, preferably within the range of about 1:1 to about 2:1 by weight, and most preferably about 65:35 by weight. In some cases, the polymeric matrix also includes a targeting molecule such as a ligand, receptor, or antibody, to increase the specificity of the microparticle for a given cell type or tissue type.

For certain applications, the microparticle has a diameter of less than about 11 microns. The microparticle can be suspended in an aqueous solution or can be in the form of a dry solid The nucleic acid can be an expression control sequence operatively linked to a coding sequence. Expression control sequences include, for example, any nucleic acid sequences known to regulate transcription or translation, such as promoters, enhancers, or silencers. In preferred examples, at least 60% or 70% of the DNA is supercoiled. More preferably, at least 80% is supercoiled.

The nucleic acid molecule (preferably in closed, circular form), may include an expression control sequence operatively linked to a coding sequence. The expression product encoded by the coding sequence can be a polypeptide at least 7 amino acids in length, having a sequence essentially identical to the sequence of either a fragment of a naturally-occurring mammalian protein or a fragment of a naturally-occurring protein from an agent which infects or otherwise harms a mammal; or a peptide having a length and sequence which permit it to bind to an MHC class I or II molecule. Examples are set forth in WO 94/04171, hereby incorporated by reference.

*Essentially identical* in the context of a DNA or polypeptide sequence is defined here to mean differing no more than 25% from the naturally occurring sequence, when the closest possible alignment is made with the reference sequence and where the differences do not adversely affect the desired function of the DNA or polypeptide in the methods of the invention. The phrase fragment of a protein is used to denote anything less than the whole protein.

To determine the percent identity of two amino acid sequences or of two nucleic acids, the sequences are aligned for optimal comparison purposes (e.g., gaps can be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences (i.e., % identity = # of identical positions/total # of positions (e.g., overlapping positions) x 100). Preferably, the two sequences are the same length.

The determination of percent homology between two sequences can be accomplished using the algorithm of Karlin and Altschul (1990) Proc. Natl. Acad. Sci. USA 87:2264-2268, modified as in Karlin and Altschul (1993) Proc. Natl. Acad Sci. USA 90:5873-5877. Such an algorithm is incorporated into the NBLAST and XBLAST programs of Altschul et al. (1990) J. Mol. Biol. 215:403-410. BLAST nucleotide searches can be performed with the NBLAST program, score = 100, wordlength = 12, to obtain nucleotide sequences homologous to a nucleic acid molecule of the invention. BLAST protein searches can be performed with the XBLAST program, score = 50, wordlength = 3, to obtain amino acid sequences homologous to a protein molecule of the invention. To obtain gapped alignments for comparison purposes, Gapped BLAST can be utilized as described in Altschul et al. (1997) Nucleic Acids Res. 25:3389-3402. Alternatively, PSI-Blast can be used to perform an iterated search which detects distant relationships between molecules. Id. When utilizing BLAST, Gapped BLAST, and PSI-Blast programs, the default parameters of the respective programs (e.g., XBLAST and NBLAST) should be used. See http://www.ncbi.nlm.nih.gov.

In calculating percent identity, only exact matches are counted.

The peptide or polypeptide can be linked to a trafficking sequence. The term "trafficking sequence" refers to an amino acid sequence which causes a polypeptide to which it is fused to be transported to a specific compartment of the cell, e.g., the nucleus, endoplasmic reticulum, the golgi apparatus, an intracellular vesicle, a lysosome, or an endosome.

In the embodiment where the expression product includes a peptide having a length and sequence which permit it to bind an MHC class I or II molecule, the expression product is typically immunogenic. The expression product can have an amino acid sequence that differs from the sequence of a naturally occurring protein recognized by a T cell in the identity of not more than 25% of its amino acid residues, provided that it can still be recognized by the same T cell and can alter the cytokine profile of the T cell (i.e., an "altered peptide ligand"). The differences between the expression product and the naturally occurring protein can, for example, be engineered to increase cross-reactivity to pathogenic viral strains or HLA-allotype binding.

Examples of expression products include amino acid sequences at least 50% identical to the sequence of a fragment of myelin basic protein (MBP), proteolipid protein (PLP), invariant chain, GAD65, islet cell antigen, desmoglein, α-crystallin, or β-crystallin, where the fragment can bind the MHC class II molecule. Table 1 lists many of such expression products that are thought to be involved in autoimmune disease. Fragments of these proteins can be essentially identical to any one of SEQ ID NOS: 1-46 such as MBP residues 80-102 (SEQ ID NO: 1), PLP residues 170-191 (SEQ ID NO: 2), or invariant chain residues 80-124 (SEQ ID NO: 3). Other fragments are listed in Table 2.

Alternatively, the expression product can include an amino acid sequence essentially identical to the sequence of an antigenic portion of any of the tumor antigens listed in Table 3 such as those encoded by the human papilloma virus E1, E2, E6 and E7 genes, Her2/neu gene, the prostate specific antigen gene, the melanoma antigen recognized by T cells (MART) gene, or the melanoma antigen gene (MAGE). Again, the expression product can be engineered to increase cross-reactivity.

In still other cases, the expression product includes an amino acid sequence essentially identical to the sequence of an antigenic fragment of a protein naturally expressed by a virus, e.g., a virus which chronically infects cells, such as human papilloma virus (HPV), human immunodeficiency virus (HIV), herpes simplex virus (HSV), hepatitis B virus (HBV), or hepatitis C virus (HCV); a bacterium, such as mycobacteria; a fungi such as Candida, *Aspergillus,* Cryptococcus, or *Histoplasmosis* species, or other eukaryotes, such as a *Plasmodium* species. A representative list of such class I-binding fragments as well as fragments of tumor antigens is included in Table 4.

The nucleic acid in the microparticles described herein can be either distributed throughout the microparticle, or can be in a small number of defined regions within the microparticle. Alternatively, the nucleic acid can be in the core of a hollow core microparticle. The microparticle preferably does not contain a cell (e.g., a bacterial cell), or a naturally occurring genome of a cell, such as a naturally occurring intact genome of a cell.

The microparticles also include a stabilizer compound, namely a carbohydrate, (other examples of a stabilizer compound include a cationic compound, a pluronic, e.g. Pluronic-F68 (Sigma-Aldrich Co., St. Louis, MO) or a DNA-condensing agent). A stabilizer compound is a compound that acts to protect the nucleic acid (e.g., to keep it supercoiled or protect it from degradation) at any time during the production of microparticles. Further examples of stabilizer compounds include dextrose, sucrose, dextran, trehalose polyvinyl alcohol, cyclodextrin, dextran sulphate, cationic peptides, pluronics, e.g. Pluronic F-68 (Sigma-Aldrich Co., St Louis, MO) and lipids such as hexadecyltrimethylammonium bromide. The stabilizer compound can remain associated with the DNA after a later release from the polymeric matrix.

The invention also feature a preparation of microparticles for use in a method of therapeutic treatment comprising administering a nucleic acid molecule to an animal by injection, the preparation comprising microparticles of the invention. In some embodiments, at least 90% of the microparticles in the preparation have a diameter less than about 100 microns. In some cases, it is desirable for at least 90% of the microparticles to have a diameter less than about 20 microns, and preferably less than about 11 microns.

In another embodiment,

**TABLE 1, Autoantigens**

| **Disease** | **Associated Antigen** | **Notes** |
|---|---|---|
| Coeliac disease | α-Gliadin | a |
| Goodpasture's syndrome collagen | Basement membrane | a |
| Graves' disease (TSH) receptor | Thyroid Stimulating Hormone | a |
| Hashimoto's disease | Thyroglobulin | a |
| Isaac's syndrome channels | voltage-gated potassium | b |
| Insulin-dependent diabetes (GAD) | Glutamic acid decarboxylase | a |
| | Insulin receptor | a |
| | Insulin associated antigen (IA-w) | a |
| | Hsp | b |
| Lambert-Eaton myasthenic calcium syndrome (LEMS) | Synaptogamin in voltage-gated channels | b |
| Multiple sclerosis | Myelin basic protein (MBP) | a |
| | Proteolipid protein (PLP) | a |
| | Myelin oligodendrocyte-associated protein (MOG) | a |
| | αB-crystallin | a |
| Myasthenia Gravis | Acetyl choline receptor | a |
| Paraneoplastic encephalitis | RNA-binding protein HuD | b |
| Pemphigus vulgaris | "PeV antigen complex" | a |
| | Desmoglein (DG) | c |
| Primary Biliary cirrhosis acetyltransferase | Dihydrolipoamide | b |
| (PDC-E2) | Pyruvate dehydrogenase complex 2 | d |
| Progressive systemic | DNA topoisomerase | a |
| sclerosis | RNA polymerase | a |
| Rheumatoid arthritis | Immunoglobulin Fc Collagen | a |
| Scleroderma | Topoisomerase I | b |
| Stiff-man syndrome (GAD) | Glutamic acid decarboxylase | a |
| Systemic lupus | erythematosus ds-DNA | a |
| Uveitis binding protein | Interphotoreceptor retinoid- | b |
| | S antigen (rod out segment) | b |

| | | |
|---|---|---|
| References: a) HLA and Autoimmune Disease, R. Heard, pg. 123-151 in HLA & Disease, Academic Press, New York, 1994, (R. Lechler, ed.) b) Cell 80, 7-10 (1995) c) Cell 67, 869-877 (1991) d) JEM 181, 1835-1845 (1995) | | |

**TABLE 2: Class II Associated Peptides**

| **Peptide Protein** | **SEQ ID NO:** | **Source** |
|---|---|---|
| GRTQDENPVVHFFKNIVTPRTPP 80-102 | 1 | MBP |
| AVYVYIYFNTWTTCQFIAFPFK 170-191 | 2 | PLP |
| FKMRMATPLLMQA chain 88-100 | 3 | Invariant |
| TVGLQLIQLINVDEVNQIV TTNVRLKQQWVDYNLKW 32-67 | 4 | Achr α |
| QIVTTNVRLKQQWVDYNLKW 67 | 5 | Achr α 48- |
| QWVDYNL 65 | 6 | Achr α 59- |
| GGVKKIHIPSEKIWRPDL 90 | 7 | Achr α 73- |
| AIVKFTKVLLQY 112 | 8 | Achr α 101- |
| WTPPAIFKSYCEIIVTHFPF 137 | 9 | Achr α 118- |
| MKLGTWTYDGSVV 156 | 10 | Achr α 144- |
| MKLGIWTYDGSVV 157 | 11 | Achr α 144-analog(I-148) |
| WTYDGSVVA 157 | 12 | Achr α 149- |
| SCCPDTPYLDITYHFVM 191-207 | 13 | Achr α |
| DTPYLDITYHFVMQRLPL 212 | 14 | Achr α 195- |
| FIVNVIIPCLLFSFLTGLVFY 234 | 15 | Achr α 214- |
| LLVIVELIPSTSS 269 | 16 | Achr α 257- |
| STHVMPNWVRKVFIDTIPN 322 | 17 | Achr α 304- |
| NWVRKVFIDTIPNIMFFS 327 | 18 | Achr α 310- |
| IPNIMFFSTMKRPSREKQ 337 | 19 | Achr α 320- |
| AAAEWKYVAMVMDHIL 410 | 20 | Achr α 395- |
| IIGTLAVFAGRLIELNQQG 437 | 21 | Achr α 419- |
| GQTIEWIFIDPEAFTENGEW 184 | 22 | Achr γ 165- |
| MAHYNRVPALPFPGDPRPYL 495 | 23 | Achr γ 476- |
| LNSKIAFKIVSQEPA 3 190-204 | 24 | desmoglein |
| TPMFLLSRNTGEVRT 3 206-220 | 25 | desmoglein |
| PLGFFPDHQLDPAFGA 10-25 | 26 | HBS preS1 |
| LGFFPDHQLDPAFGANS preS1 11-27 | 27 | HBS |
| FFLLTRILTI 28 | 28 | HBS Ag 19- |
| RILTIPQSLD 33 | 29 | HBS Ag 24- |
| TPTLVEVSRNLGK | 30 | HSA 444-456 |
| AKTIAYDEEARR | 31 | hsp 65 2-13 |
| VVTVRAERPG 70 | 32 | hsp 18 61- |
| SQRHGSKYLATASTMDHARHG | 33 | MBP 7-27 |
| RDTGILDSIGRFFGGDRGAP | 34 | MBP 33-52 |
| QKSHGRTQDENPVVHFFKNI | 35 | MBP 74-93 |
| DENPVVHFFKNIVT | 36 | MBP 84-97 |
| ENPVVHFFKNIVTPR | 37 | MBP 85-99 |
| HFFKNIVTPRTPP | 38 | MBP 90-102 |
| KGFKGVDAQGTLSK | 39 | MBP 139-152 |
| VDAQGTLSKIFKLGGRDSRS | 40 | MBP 144-163 |
| LMQYIDANSKFIGITELKK Toxoid 828-846 | 41 | Tetanus |
| QYIKANSKFIGIT Toxoid 830-842 | 42 | Tetanus |
| FNNFTVSFWLRVPK Toxoid 947-960 | 43 | Tetanus |
| SFWLRVPKVSASHLE Toxoid 953-967 | 44 | Tetanus |
| KFIIKRYTPNNEIDSF Toxoid 1174-1189 | 45 | Tetanus |
| GQIGNDPNRDIL Toxoid 1273-1284 | 46 | Tetanus |

**TABLE 3: Tumor Antigens**

| **Cancer** | **Associated Antigen** |
|---|---|
| Melanoma Breast/Ovarian (Her2/neu) | BAGE 2-10 c-ERB2 |
| Burkitt's lymphoma/Hodgkin's lymphoma | EBNA-1 |
| Burkitt's lymphoma/Hodgkin's lymphoma | EBNA-2 |
| Burkitt's lymphoma/Hodgkin's lymphoma | EBNA-3 |
| Burkitt's lymphoma/Hodgkin's | lymphoma EBNA-3A |
| Burkitt's lymphoma/Hodgkin's lymphoma | EBNA-3C |
| Burkitt's lymphoma/Hodgkin's lymphoma | EBNA-4 |
| Burkitt's lymphoma/Hodgkin's lymphoma | EBNA-6 |
| Burkitt's lymphoma/Hodgkin's lymphoma | EBV |
| Burkitt's lymphoma/Hodgkin's lymphoma | EBV LMP2A |
| Melanoma | GAGE-1 |
| Melanoma | gp75 |
| Cervical | HPV 16 E6 |
| Cervical | HPV 16 E7 |
| Cervical | HPV 18 E6 |
| Cervical | HPV 18 E7 |
| Melanoma | MAG |
| Melanoma | MAGE-1 |
| Melanoma | MAGE-2 |
| Melanoma | MAGE-3 |
| Melanoma | MAGE-4b |
| Melanoma | MAGE-5 |
| Melanoma | MAGE-6 |
| Melanoma | MART-1/Melan-A |
| Pancreatic/Breast/Ovarian | MUC-1 |
| Melanoma | MUM-1-B |
| Breast/Colorectal/Burkitt's lymphoma | p53 |
| Melanoma | Pmel 17(gp100) |
| Prostate | PSA Prostate |
| Specific Antigen | |
| Melanoma | Tyrosinase CEA Carcinoembryonic |
| Antigen | LRP Lung Resistance |
| Protein | Bcl-2 |
| | Ki-67 |

**TABLE 4: Class I associated tumor and pathogen peptides**

| **Peptide Protein** | **SEQ ID NO:** | **Source** |
|---|---|---|
| AARAVFLAL | 47 | BAGE 2-10 |
| YRPRPRRY | 48 | GAGE-1 9-16 |
| EADPTGHSY | 49 | MAGE-1 161-169 |
| SAYGEPRKL 238 | 50 | MAGE-1 230- |
| EVDPIGHLY 169 | 51 | MAGE-3 161- |
| FLWGPRALV 279 | 52 | MAGE-3 271- |
| GIGILTV 35 | 53 | MART-1 29- |
| ILTVILGV 39 | 54 | MART-1 32- |
| STAPPAHGV | 55 | MUC-1 9-17 |
| EEKLIVVLF 269 | 56 | MUM-1 261- |
| MLLAVLYCL 1-9 | 57 | TYROSINASE |
| SEIWRDIDF 192-200 | 58 | TYROSINASE |
| AFLPWHRLF 206-214 | 59 | TYROSINASE |
| YMNGTMSQV 369-376 | 60 | TYROSINASE |
| KTWGQYWQV (GP100) 154-162 | 61 | PMEL 17 |
| ITDQVPFSV (GP100) 209-217 | 62 | PMEL 17 |
| YLEPGPTVA (GP100) 280-288 | 63 | PMEL 17 |
| LLDGTATLRL (GP100) 476-485 | 64 | PMEL 17 |
| ELNEALELEK | 65 | p53 343-351 |
| STPPPGTRV | 66 | p53 149-157 |
| LLPENNVLSPL | 67 | p53 25-35 |
| LLGRNSFEV | 68 | p53 264-272 |
| RMPEAAPPV | 69 | p53 65-73 |
| KIFGSLAFL 369-377 | 70 | HER-2/neu |
| IISAVVGIL 654-662 | 71 | HER-2/neu |
| CLTSTVQLV 789-797 | 72 | HER-2/neu |
| YLEDVRLV 835-842 | 73 | HER-2/neu |
| VLVKSPNHV 851-859 | 74 | HER-2/neu |
| RFRELVSEFSRM 968-979 | 75 | HER-2/neu |
| LLRLSEPAEL | 76 | PSA 119-128 |
| DLPTQEPAL | 77 | PSA 136-144 |
| KLQCVD | 78 | PSA 166-171 |
| VLVASRGRAV | 79 | PSA 36-45 |
| VLVHPQWVL | 80 | PSA 49-57 |
| DMSLLKNRFL | 81 | PSA 98-107 |
| QWNSTAFHQ envelope 121-130 | 82 | HBV |
| VLQAGFF envelope 177-184 | 83 | HBV |
| LLLCLIFL envelope 250-257 | 84 | HBV |
| LLDYQGML envelope 260-267 | 85 | HBV |
| LLVPFV 338-343 | 86 | HBV envelope |
| SILSPFMPLL envelope 370-379 | 87 | HBV |
| PLLPIFFCL envelope 377-385 | 88 | HBV |
| ILSTLPETTV 529-538 | 89 | HBV core |
| FLPSDFFPSV 47-56 | 90 | HBV core |
| KLHLYSHPI polymerase 489-498 | 91 | HBV |
| ALMPLYACI polymerase 642-651 | 92 | HBV |
| HLYSHPIIL 1076- | 93 | HBV polym. 1084 |
| FLLSLGIHL 1147- | 94 | HBV polym. 1153 |
| HLLVGSSGL polymerase 43-51 | 95 | HBV |
| GLSRYVARL polymerase 455-463 | 96 | HBV |
| LLAQFTSAI polymerase 527-535 | 97 | HBV |
| YMDDVVLGA polymerase 551-559 | 98 | HBV |
| GLYSSTVPV polymerase 61-69 | 99 | HBV |
| NLSWL 996-1000 | 100 | HBV polymerase |
| KLPQLCTEL 18-26 | 101 | HPV 16 E6 |
| LQTTIHDII 26-34 | 102 | HPV 16 E6 |
| FAFRDLCIV 52-60 | 103 | HPV 16 E6 |
| YMLDLQPET 11-19 | 104 | HPV 16 E7 |
| TLHEYMLDL 7-15 | 105 | HPV 16 E7 |
| LLMGTLGIV 82-90 | 106 | HPV 16 E7 |
| TLGIVCPI 86-93 | 107 | HPV 16 E7 |
| LLMGTLGIVCPI 82-93 | 108 | HPV 16 E7 |
| LLMGTLGIVCPICSQK | 109 | HPV 16 E7 82-97 |

the nucleic acid molecule includes an expression control sequence operatively linked to a coding sequence. The expression product encoded by the coding sequence is a protein which, when expressed in a macrophage *in vivo,* downregulates an immune response, either specifically or in general. Examples of such proteins include tolerizing proteins, MHC blocking peptides, altered peptide ligands, receptors, transcription factors, and cytokines.

In some embodiments of the microparticles described herein, the nucleic acid need not encode a peptide, but could modulate an immune response by stimulating the release of γ-interferon, IL-12, or other cytokines, or by polyclonally activating B cells, macrophages, dendritic cells, or T cells. For example, poly I:C or CpG-containing nucleic acid sequences can be used (Klinman et al., Proc. Nat. Acad. Sci. (USA) 93:2879, 1996; Sato et al., Science 273:352, 1995).

Also disclosed herein is a process for preparing microparticles. A first solution, including a polymer dissolved in an organic solvent, is mixed (e.g., sonication, homogenization, vortexing, or microfluidization) with a second solution, which includes a nucleic acid dissolved or suspended in a polar or hydrophilic solvent (e.g., an aqueous buffer solution containing, for instance, ethylenediaminetetraacetic acid, or tris(hydroxymethyl)aminomethane, or combinations thereof). The mixture forms a first emulsion. The first emulsion is then mixed with a third solution which can include a surfactant such as Pluronic, e.g., Pluronic F-68 (Sigma-Aldrich Co.), to form a second emulsion containing microparticles of polymer matrix and nucleic acid. The mixing steps can be executed, for example, in a homogenizer, vortex mixer, microfluidizer, or sonicator. Both mixing steps are carried out in a manner that minimizes shearing of the nucleic acid while producing microparticles on average smaller than 100 microns in diameter.

The second solution can, for example, be prepared by column chromatography and further purification of the nucleic acid (e.g., by ethanol or isopropanol precipitation), then dissolving or suspending the purified or precipitated nucleic acid in an aqueous, polar, or hydrophilic solution.

The first or second solution can optionally include a surfactant, a DNA-condensing agent, or a stabilizer compound (e.g., 1-10% dextrose, trehalose, sucrose, dextran, or other carbohydrates, polyvinyl alcohol, cyclodextrin, hexadecyltrimethylammonium bromide, Pluronic F-68 (Sigma-Aldrich Co.,, St. Louis, MO), another lipid, or dextran sulfate) that can stabilize the nucleic acid or emulsion by keeping the nucleic acid supercoiled during encapsulation and throughout the microparticle formation.

The second emulsion is optionally mixed with a fourth solution including an organic solvent. The second emulsion can optionally be stirred (i.e., alone or as a mixture with the fourth solution) at an elevated temperature (e.g., room temperature to about 60°C), for example, to facilitate more rapid evaporation of the solvents. Alternative ways to remove solvent include addition of alcohol, application of a vacuum, or dilution.

The procedure can include the additional step of washing the microparticles with an aqueous solution to remove organic solvent, thereby producing washed microparticles. The washed microparticles can then be subjected to a temperature below 0°C, to produce frozen microparticles, which are in turn lyophilized to produce lyophilized microparticles. The microparticles can optionally be suspended in water or in an excipient, such as Tween-80, mannitol, sorbitol, or carboxymethylcellulose, prior to or after lyophilization (if any).

When desired, the procedure can include the additional step of screening the microparticles to remove those larger than 100 microns (or even 20 microns) in diameter.

Also disclosed herein is a preparation of microparticles which include a polymeric matrix, a proteinaceous antigenic determinant, and a DNA molecule which encodes an antigenic polypeptide that can be different from, or the same as, the aforementioned proteinaceous antigen determinant. The antigenic determinant contains an epitope which can elicit an antibody response. The antigenic polypeptide expressed from the DNA can induce a T cell response (e.g., a CTL response). The DNA can be plasmid DNA, and can be combined in the same microparticle as the antigenic determinant, or the two can be in distinct microparticles which are then mixed together. In some case, an oligonucleotide, rather than a proteinaceous antigenic determinant, can be encapsulated together with a nucleic acid plasmid. Alternatively, the oligonucleotide may be encapsulated in a separate particle. The oligonucleotide can have antisense or ribozyme activity, for example.

In accordance with the invention, the microparticle is for use in a method of therapeutic treatment comprising administering the nucleic acid to the animal by introducing into the animal (e.g., a mammal such as a human, non-human primate, horse, cow, pig, sheep, goat, dog, cat, mouse, rat, guinea, hamster, or ferret) the microparticle of the invention. The microparticles can be provided suspended in an aqueous solution or any other suitable formulation. They can optionally be delivered in a conjunction with a protein such as a cytokine, an interferon, an antigen, or an adjuvant.

In another embodiment, with regard to the preparation of microparticles, the polymeric matrix includes one or more synthetic polymers having a solubility in water of less than about 1mg/l; in the present context, synthetic is defined as non-naturally occurring. At least 90% of the microparticles have a diameter less than about 100 microns. The nucleic acid can be either RNA or DNA. When present as RNA, in some embodiments at least 50% (and preferably at least 70% or even 80%) is in the form of closed circles. The nucleic acid can be linear or circular molecule, and can thus be, e.g., a plasmid, or may include a viral genome, or part of a viral genome. When circular and double-stranded, it can be nicked, i.e., in an open circle, or super-coiled. In some embodiments the nucleic acids are plasmid molecules at least 25%, (and preferably at least 35%, 40%, 50%, 60%, 70%, or even 80%) of which are supercoiled.

The nucleic acid can also be oligonucleotide, e.g., an antisense oligonucleotide or ribozyme. The preparation also includes a stabilizer compound namely a carbohydrate. Other stabilizer compounds include+ dextrose, sucrose, dextran, trehalose polyvinyl alcohol, cyclodextrin, dextran sulphate, and cationic peptides.

Also disclosed herein is a preparation of microparticles, each of which comprises a polymeric matrix, a nucleic acid molecule, and a lipid. The microparticles are not encapsulated in liposomes, and the microparticles do not comprise cells. By "do not comprise cells" is meant that the microparticles do not contain cells (e.g., bacterial cells). It is understood that the microparticles may themselves be taken up by cells such as macrophages, as is explained above.

The nucleic acid may be any of the above-mentioned nucleic acid molecules and may also include an isolated nucleic molecule. By isolated nucleic acid molecule is meant any synthetic (including recombinant) nucleic acid molecule or a naturally occurring nucleic acid molecule removed from the virus or cell in which it is normally present.

The lipid can be, e.g, a cationic lipid, an anionic lipid, or a zwitterionic lipid, or may have no charge. Examples of lipids include cetyltrimethylammonium and phospholipids, e.g., phosphatidylcholine. The microparticles may contain one or more than one type of lipid, e.g., those lipids present in lecithin lipid preparations, and may also include one or more stabilizer compounds as described above.

Also disclosed herein is a microparticle less than about 20 microns in diameter, which includes a polymeric matrix, a lipid, and a nucleic acid molecule. The microparticle is not encapsulated in a liposome, and the microparticle does not comprise a cell.

The nucleic acid molecule in the microparticle can be circular, and the nucleic acid molecule may include an expression control sequence operatively linked to a coding sequence. The microparticle may optionally include a stabilizer compound or targeting molecule as described above. Also disclosed herein is a

microparticle less than about 20 microns in diameter which preferably is not encapsulated in a liposome. The microparticle includes a polymeric matrix, a lipid, and a nucleic acid molecule that includes an expression control sequence operatively linked to a coding sequence. The coding sequence encodes an expression product that can include: (1) a polypeptide at least 7 amino acids in length, having a sequence essentially identical to the sequence of (a) a fragment of a naturally-occurring mammalian protein, or (b) a fragment of a naturally-occurring protein from an infectious agent which infects a mammal; (2) a peptide having a length and sequence which permit it to bind to an MHC class I or II molecule; and the polypeptide or peptide linked to a trafficking sequence. The expression product can additionally include an amino terminal methionine residue, and can also be immunogenic.

The expression product may include overlapping antigenic peptides derived from (1) (a) or (1) (b) or (2) above, e.g., two, three, four or more antigenic peptides arranged in series, where the sequence at the carboxy terminal end of the first forms a portion of the amino terminal end of the second, and a portion of the carboxy terminal end of the second forming a portion of the amino terminal end of the third, etc. An example of an amino acid sequence containing overlapping peptides is the amino acid sequence LLMGTLGIVCPIC (SEQ ID NO:110), which includes the MHC class I-binding peptides LLMGTLGIV (SEQ ID NO:111) and TLGIVCPIC (SEQ ID NO:115).

The expression product may alternatively or in addition include a polypeptide having two or more antigenic peptides, wherein the antigenic regions do not overlap. These tandem arrays of peptides may include two, three, four or more peptides (e.g., up to ten or twenty or more) which can be the same or different. Such tandemly arranged peptides can, of course, be interspersed with overlapping peptides.

In some embodiments, the expression product (1) has an amino acid sequence that differs by no more than 25% from the sequence of a naturally occurring peptide recognized by a T cell; (2) is recognized by the T cell; and preferably (3) alters the cytokine profile of the T cell (e.g., an "altered peptide ligand").

The above expression product may include an MHC class II-binding amino acid sequence at least 50% identical to the sequence of a fragment of a protein at least 10 amino acids in length. The protein can be, e.g., myelin basic protein (MBP), proteolipid protein (PLP), invariant chain, GAD65, islet cell antigen, desmoglein, α-crystallin, or β-crystallin, or may be an amino acid sequence essentially identical to one or more of the sequences of SEQ ID NOS 1-46.

The above expression product can also include a trafficking sequence, e.g., a sequence that trafficks to endoplasmic reticulum, a sequence which trafficks to a lysosome, a sequence which trafficks to an endosome, a sequence which trafficks to an intracellular vesicle, or a sequence which trafficks to the nucleus. Such trafficking sequences include signal peptides (the amino terminal sequences which direct proteins into the ER during translation), ER retention peptides such as KDEL, and lysosome-targeting peptides such as KFERQ and QREFK, and other pentapeptides having Q flanked on one side by four residues selected from K, R, D, E, F, I, V, and L. Nuclear localization sequences include nucleoplasmin- and SV40-like nuclear targeting signals as described in Chelsky et al., Mol. Cell Biol., 9_:2487, 1989; Robbins, Cell, 64 :615, 1991, and Dingwall et al., TIBS, 16:478, 1991. Some nuclear localization sequences include AVKRPAATKKAGQAKKK (SEQ ID NO:112), RPAATKKAGQAKKKKLD (SEQ ID NO:123), and AVKRPAATKKAGQAKKKLD (SEQ ID NO:114).

The expression product can include an amino acid sequence essentially identical to the sequence of an antigenic portion of a tumor antigen, e.g., a tumor antigen from one of the proteins listed in Table 3.

The expression product may also include an amino acid sequence essentially identical to the sequence of an antigenic fragment of a protein naturally expressed by an infectious agent. The infectious agent can be, e.g., virus, a bacterium, or a parasitic eukaryote, e.g., a yeast. The infectious agent can thus include, e.g., human papilloma virus, human immunodeficiency virus, herpes simplex virus, hepatitis B virus, hepatitis C virus, *Plasmodium* species, mycobacteria, *Chlamydia,* and *Helicobacter* species.

Also disclosed herein is a method of administering a nucleic acid to an animal (e.g., a human) by introducing the lipid-containing microparticles described above into the animal. The lipid particles may in addition include stabilizing agents. The microparticles may be introduced via oral, mucosal, inhalation, or parenteral routes, e.g., by subcutaneous, intramuscular, or intraperitoneal injection.

Also disclosed herein is a process for preparing lipid-containing microparticles. The steps include providing a first solution which contains a polymer dissolved in an organic solvent, and providing a second solution which includes a nucleic acid dissolved or suspended in a polar or hydrophilic solvent. The first and second solutions are mixed to form a first emulsion. The first emulsion is then mixed with a third solution to form a second emulsion. At least one of the first, second and third solutions also includes a lipid or lipids. Both mixing steps are carried out in a manner that minimizes shearing of the nucleic acid while producing microparticles having an average diameter smaller than 100 microns.

The lipid or lipids can be included in either the first, second, or third solution, or in a combination of these solutions. In some embodiments the lipid is present in a concentration of 0.001 to 10.0%, or 0.1 to 1.0% (weight/volume), in one or more of the solutions.

The process may optionally include subjecting the microparticles to a temperature below 0°C, to produce frozen microparticles, and lyophilizing the frozen microparticles, to produce lyophilized microparticles.

Also disclosed herein is a preparation of microparticles, each of which includes a polymeric matrix, a lipid, a proteinaceous antigenic determinant, an isolated nucleic acid molecule which encodes an antigenic polypeptide, and, optionally, a stabilizer agent.

Also disclosed herein is a method of administering nucleic acid to an animal by providing a preparation of lipid-containing microparticles and introducing the preparation into the animal. The lipid-containing microparticles may optionally contain at least one stabilizer agent, e.g., a carbohydrate.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, the preferred methods and materials are described below.

In case of conflict, the present specification, including definitions, will control. In addition, the materials, methods, and examples are illustrative only and not intended to be limiting.

Other features and advantages of the invention will be apparent from the following detailed description, and from the claims.

### Brief Description of the Drawings

Figs. 1A to 1C are a set of three plasmid maps, of the pvA2.1/4, luciferase, and VSV-Npep plasmids, respectively.
Fig. 2 is a plot of size distribution of DNA-containing microparticles as analyzed on a COULTER^{™} counter.
Figs. 3A and 3B are a set of photographs of two agarose electrophoresis gels indicating degree of DNA supercoiling as a function of different homogenization speeds and durations.
Figs. 4A and 4B are a pair of FACS printouts comparing cell populations in the absence or presence of microparticles.
Figs. 5 to 9 are plots of specific lysis versus effector:target ratio.
Fig. 10 is a graph showing the release over time of DNA from microparticles prepared from DNA resuspended in TE or CTAB.
Fig. 11 is a graph showing the release over time of DNA from microparticles containing no lipid ("TE"), lecithin, or OVOTHIN^{™} 160.
Fig. 12 is a graph showing T cell responses from mice injected with lipid-containing microparticles containing luciferase-encoding DNA.

### Detailed Description

The microparticles disclosed herein are formulated in one of two ways: (1) to maximize delivery into the patient's phagocytic cells, or (2) to form a deposit in the tissues of the patient, from which the nucleic acid is released gradually over time; upon release from the microparticle, the nucleic acid is taken up by neighboring cells (including antigen presenting cells, or APCs). In both cases, maintaining the integrity of the DNA is a priority. For plasmid DNA, this means maximizing the percentage of plasmid molecules that are supercoiled and which may be capable of more efficient transfection and transcription than non-supercoiled (i.e., nicked or linear) plasmids. Maximizing the percentage of supercoiled plasmid molecules may also increase the stability of the DNA in the cell or microparticle.

Means for protecting the integrity of the nucleic acid include minimizing the shearing forces to which the nucleic acid is necessarily exposed in the process of microparticle formation, limiting sonication, homogenization, microfluidization, or other mixing times during preparation, and adding buffers or other stabilizer compounds during nucleic acid isolation and microparticle preparation. For example, it is desirable to achieve a balance between sonication time and intensity which minimizes shear yet produces the desired size of microparticles. These techniques are discussed below.

The microparticles disclosed herein can be used in the manufacture of a medicament for the treatment of, for example, cancer, any of the autoimmune diseases listed in Table 1, or any other condition treatable with a particular defined nucleic acid.

Phagocytosis of microparticles by macrophages, dendritic cells, and other APCs is an effective means for introducing the nucleic acid into these cells. Phagocytosis by these cells can be increased by maintaining a particle size below about 20 µm, and preferably below about 11 µm. The type of polymer used in the microparticle can also affect the efficiency of uptake by phagocytic cells, as discussed below.

The microparticles can be delivered directly into the bloodstream (i.e., by intravenous or intraarterial injection or infusion) where uptake by the phagocytic cells of the reticuloendothelial system (RES) is desired. Alternatively, the microparticles can be delivered orally, into mucosally sites, nasally, vaginally, rectally or intralesionally. The microparticles can also be delivered via subcutaneous injection, to facilitate take-up by the phagocytic cells of the draining lymph nodes. Alternatively, the microparticles can be introduced intradermally (i.e., to the APCs of the skin, such as dendritic cells and Langerhans cells) or intramuscularly. Finally, the microparticles can be introduced into the lung (e.g., by inhalation of powdered microparticles or of a nebulized or aerosolized solution or suspension containing the microparticles), where the particles are picked up by the alveolar macrophages.

Once a phagocytic cell phagocytoses the microparticle, the nucleic acid is released into the interior of the cell. Upon release, it can perform its intended function: for example, expression by normal cellular transcription/translation machinery (for an expression vector), or alteration of cellular processes (for antisense or ribozyme molecules).

Because these microparticles are passively targeted to macrophages and other types of professional APC and phagocytic cells, they represent a means for modulating immune function. Macrophages and dendritic cells serve as professional APCs, expressing both MHC class I and class II molecules. In addition, the mitogenic effect of DNA can be used to stimulate non-specific immune responses mediated by B, T, NK, and other cells.

Delivery, via microparticles, of an expression vector encoding a foreign antigen which binds to an MHC class I or class II molecule will induce a host T cell response against the antigen, thereby conferring host immunity.

Where the expression vector encodes a blocking peptide (See, e.g., WO 94/04171) that binds to an MHC class II molecule involved in autoimmunity, presentation of the autoimmune disease-associated self peptide by the class II molecule is prevented, and the symptoms of the autoimmune disease alleviated.

An MHC binding peptide that is identical or almost identical to an autoimmunity-inducing peptide can affect T cell function by tolerizing or anergizing the T cell. Alternatively, the peptide could be designed to modulate T cell function by altering cytokine secretion profiles following recognition of the MHC/peptide complex. Peptides recognized by T cells can induce secretion of cytokines that cause B cells to produce antibodies of a particular class, induce inflammation, and further promote host T cell responses.

Induction of immune responses, e.g., specific antibody responses to peptides or proteins, can require several factors. It is this multifactorial nature that provides impetus for attempts to manipulate immune related cells on multiple fronts, using the microparticles disclosed herein. For example, microparticles can be prepared which carry both DNA and polypeptides within each microparticle; alternatively, microparticles can be prepared which carry either DNA or polypeptide, and then mixed. These dual-function microparticles are discussed below.

### CTL Responses

Class I molecules present antigenic peptides to immature T cells. To fully activate T cells, factors other than the antigenic peptide are required. Non-specific proteins such as interleukin-2 (IL-2), IL-12, and gamma interferon (γ-IFN) promote CTL responses and can be provided together with DNA encoding polypeptides which include CTL epitopes. Alternatively, proteins which bear helper T (T_{H}) determinants can be included with DNA encoding the CTL epitope. T_{H} epitopes promote secretion of cytokines from T_{H} cells and play a role in the differentiation of nascent T cells into CTLs.

Alternatively, proteins, nucleic acids, or adjuvants which promote migration of lymphocytes and macrophages to a particular area could be included in microparticles along with appropriate DNA molecules. Uptake of the DNA is enhanced as a result, because release of the protein would cause an influx of phagocytic cells and T cells as the microparticle degrades. The macrophages would phagocytose the remaining microparticles and act as APC, and the T cells would become effector cells.

### Antibody Responses

Elimination of certain infectious agents from the host may require both antibody and CTL responses. For example, when the influenza virus enters a host, antibodies can often prevent it from infecting host cells. However, if cells are infected, then a CTL response is required to eliminate the infected cells and to prevent the continued production of virus within the host.

In general, antibody responses are directed against conformational determinants and thus require the presence of a protein or a protein fragment containing such a determinant. In contrast, T cell epitopes are linear determinants, typically just 7-25 residues in length. Thus, when there is a need to induce both a CTL and an antibody response, the microparticles can include both an antigenic protein and the DNA encoding a T cell epitope.

Slow release of the protein from microparticles would lead to B cell recognition and subsequent secretion of antibody, while phagocytosis of the microparticles would cause APCs (1) to express the DNA of interest, thereby generating a T cell response; and (2) to digest the protein released from the microparticles, thereby generating peptides which are subsequently presented by class I or II molecules. Presentation by class I or II molecules promotes both antibody and CTL responses, since T_{H} cells activated by the class II/peptide complexes would secrete non-specific cytokines.

### Immunosuppression

Certain immune responses lead to allergy and autoimmunity, and so can be deleterious to the host. In these instances, there is a need to inactivate tissue-damaging immune cells. Immunosuppression can be achieved with microparticles bearing DNA that encodes epitopes that down-regulate T_{H} cells or CTLs, e.g., blocking peptides and tolerizing peptides. In these microparticles, the effect of the immunosuppressive DNA could be amplified by including certain proteins in the carrier microparticles with the DNA. A list of such proteins includes antibodies, receptors, transcription factors, and the interleukins.

For example, antibodies to stimulatory cytokines or homing proteins, such as integrins or intercellular adhesion molecules (ICAMs), can increase the efficacy of the immunosuppressive DNA epitope. These proteins serve to inhibit the responses of already-activated T cells, while the DNA further prevents activation of nascent T cells. Induction of T cell regulatory responses can be influenced by the cytokine milieu present when the T cell receptor (TCR) is engaged. Cytokines such as IL-4, IL-10, and IL-6 promote T_{H}2 differentiation in response to the DNA-encoded epitope. T_{H}2 responses can inhibit the activity of T_{H}1. cells and the corresponding deleterious responses which result in the pathologies of rheumatoid arthritis, multiple sclerosis and juvenile diabetes.

Inclusion of proteins comprising soluble forms of costimulatory molecules (e.g., CD-40, gp-39, B7-1, and B7-2), or molecules involved in apoptosis (e.g., Fas, FasL, Bc12, caspase, bax, TNFα, or TNFα receptor) is another way to inhibit activation of particular T cell and/or B cells responses. For example, B7-1 is involved in the activation of T_{H}1 cells, and B7-2 activates T_{H}2 cells. Depending on the response that is required, one or the other of these proteins could be included in the microparticle with the DNA, or could be supplied in separate microparticles mixed with the DNA-containing microparticles.

### Microparticles for Implantation

A second microparticle formulation disclosed herein is intended not to be taken up directly by cells, but rather to serve primarily as a slow-release reservoir of nucleic acid that is taken up by cells only upon release from the microparticle through biodegradation. The nucleic acid can be complexed to a stabilizer, e.g., to maintain the integrity of the nucleic acid during the slow-release process. There polymeric particles should therefore be large enough to preclude phagocytosis (i.e., larger than 5 µm and preferably larger than 20 µm). Such particles are produced by the methods described above for making the smaller particles, but with less vigorous mixing of the aforementioned first or second emulsions. That is to say, a lower homogenization speed, vortex mixing speed, or sonication setting can be used to obtain particles having a diameter around 100 µm rather than 5 µm. The time of mixing, the viscosity of the first emulsion, or the concentration of polymer in the first solution can also be altered to affect particle dimension.

The larger microparticles can be formulated as a suspension, a powder, or an implantable solid, to be delivered by intramuscular, subcutaneous, intradermal, intravenous, or intraperitoneal injection; via inhalation (intranasal or intrapulmonary); orally, e.g. in the form of a tablet; or by implantation. These particles are useful for delivery of any expression vector or other nucleic acid for which slow release over a relatively long term is desired: e.g., an antisense molecule, a gene replacement therapeutic, a means of delivering cytokine-based, antigen-based, or hormone-based therapeutic, or an immunosuppressive agent. The rate of degradation, and consequently of release, varies with the polymeric formulation. This parameter can be used to control immune function. For example, one would want a relatively slow release for delivery of IL-4 or IL-10, and a relatively rapid release for delivery of IL-2 or γ-IFN.

### Composition of Polymeric Particles

Polymeric material is obtained from commercial sources or can be prepared by known methods. For example, polymers of lactic and glycolic acid can be generated as described in US Patent No. 4,293,539 or purchased from Aldrich.

Alternatively, or in addition, the polymeric matrix can include polylactide, polyglycolide, poly(lactide-co-glycolide), polyanhydride, polyorthoester, polycaprolactone, polyphosphazene, proteinaceous polymer, polypeptide, polyester, or naturually occurring polymers such as alginate, chitosan, and gelatin.

Preferred controlled release substances which are useful in the formulations of the invention include the polyanhydrides, co-polymers of lactic acid and glycolic acid wherein the weight ratio of lactic acid to glycolic acid is no more than 4:1, and polyorthoesters containing a degradation-enhancing catalyst, such as an anhydride, e.g., 1% maleic anhydride. Since polylactic acid can take at least one year to degrade *in vivo*, this polymer should be utilized by itself only in circumstances where extended degradation is desirable.

### Association of Nucleic Acid and Polymeric Particles

Polymeric particles containing nucleic acids can be made using a double emulsion technique. First, the polymer is dissolved in an organic solvent. A preferred polymer is polylactic-co-glycolic acid (PLGA), with a lactic/glycolic acid weight ratio of 65:35, 50:50, or 75:25. Next, a sample of nucleic acid suspended in aqueous solution is added to the polymer solution and the two solutions are mixed to form a first emulsion. The solutions can be mixed by vortexing, microfluidization, shaking, sonication, or homogenization. Most preferable is any method by which the nucleic acid receives the least amount of damage in the form of nicking, shearing, or degradation, while still allowing the formation of an appropriate emulsion. For example, acceptable results can be obtained with a Vibra-cell model VC-250 sonicator with a 1/8" microtip probe, at setting #3, or by controlling the pressure in the microfluidizer, or by using an SL2T Silverson Homogenizer with a 5/8" tip at 10K.

During this process, water droplets (containing the nucleic acid) form within the organic solvent. If desired, one can isolate a small amount of the nucleic acid at this point in order to assess integrity, e.g., by gel electrophoresis.

Alcohol precipitation or further purification of the nucleic acid prior to suspension in the aqueous solution can improve encapsulation efficiency. Precipitation with ethanol resulted in up to a 147% increase in incorporated DNA and precipitation with isopropanol increased incorporation by up to 170%.

The nature of the aqueous solution can affect the yield of supercoiled DNA. For example, the presence of detergents such as polymyxin B, which are often used to remove endotoxins during the preparation and purification of DNA samples, can lead to a decrease in DNA encapsulation efficiency. It may be necessary to balance the negative effects on encapsulation efficiency with the positive effects on supercoiling, especially when detergents, surfactants, and/or stabilizers are used during encapsulation. Furthermore, addition of buffer solutions containing either tris(hydroxymethyl)aminomethane (TRIS), ethylenediaminetetraacetic acid (EDTA), or a combination of TRIS and EDTA (TE) resulted in stabilization of supercoiled plasmid DNA, according to analysis by gel electrophoresis. Ph effects are also observed. Other stabilizing compounds, such as dextran sulfate, dextrose, dextran, CTAB, polyvinyl alcohol, and sucrose, were also found to enhance the stability and degree of supercoiling of the DNA, either alone or in combination with the TE buffer. Combinations of stabilizers can be used to increase the amount of supercoiled DNA. Stabilizers such as charged lipids (e.g., CTAB), pluronics, e.g., Pluroinc F-68 (Sigma-Aldrich Co., St. Louis, MO), cationic peptides, or dendrimers (J. Controlled Release, 39:357, 1996) can condense or precipitate the DNA. Moreover, stabilizers can have an effect on the physical nature of the particles formed during the encapsulation procedure. For example, the presence of sugars or surfactants during the encapsulation procedure can generate porous particles with porous interior or exterior structures, allowing for a more rapid exit of a drug from the particle. The stabilizers can act at any time during the preparation of the microspheres: during encapsulation or lyophilization, or both, for example.

The first emulsion is then added to an organic solution, allowing formation of microparticles. The solution can be comprised of, for example, methylene chloride, ethyl acetate, acetone, polyvinyl pyrrolidone (PVP) and preferably contains polyvinyl alcohol (PVA). Most preferably, the solution has a 1:100 to 8:100 ratio of the weight of PVA to the volume of the solution. The first emulsion is generally added to the organic solution with stirring in a homogenizer (e.g., a Silverson Model L4RT homogenizer (5/8" probe) set at 7000 RPM for about 12 seconds) or a microfluidizer.

This process forms a second emulsion which is subsequently added to another organic solution with stirring (e.g., in a homogenizer, microfluidizer, or on a stir plate). This step causes the first organic solvent (e.g., dichloromethane) to be released and the microspheres to become hardened. Heat, vacuum, or dilution can alternatively be used to accelerate evaporation of the solvent. Slow release of the organic solvent (e.g., at room temperature) can result in "spongy" particles, while fast release (e.g., at elevated temperature) results in hollow-core microparticles. The latter solution can be, for example, 0.05% w/v PVA. If sugar or other compounds are added to the DNA, an equal concentration of the compound can be added to the third or fourth solution to equalize osmolarity, effectively decreasing the loss of nucleic acid from the microsphere during the hardening process. The resultant microparticles are washed several times with water to remove the organic compounds. Particles can be passed through sizing screens to selectively remove those larger than the desired size. If the size of the microparticles is not crucial, one can dispense with the sizing step. After washing, the particles can either be used immediately, frozen for later use, or be lyophilized for storage.

Larger particles, such as those used for implantation, can be obtained by using less vigorous emulsification conditions when making the first emulsion, as has already been described above at length. For example, larger particles can also be obtained by altering the concentration of the polymer, altering the viscosity of the emulsion, altering the particle size of the first emulsion (e.g., larger particles can be made by decreasing the pressure used while creating the first emulsion in a microfluidizer), or homogenizing with, for example, the Silverson homogenizer set at 5000 RPM for about 12 seconds.

The washed, or washed and lyophilized, microparticles can be suspended in an excipient without negatively affecting the amount of supercoiled plasmid DNA within the microspheres. Excipients such as carbohydrates, polymers, or lipids are often used in drug formulation, and here provide for efficient microsphere resuspension, act to prevent settling, and/or retain the microspheres in suspension. According to analysis by gel electrophoresis, excipients (including Tween 80, mannitol, sorbitol, and carboxymethylcellulose) have no effect on DNA stability or supercoiling, when included prior to or after lyophilization.

After recovery of the microspheres or suspension of the microspheres in an excipient, the samples can be frozen and lyophilized for future use.

### Characterization of Microparticles

The size distribution of the microparticles prepared by the above method can be determined with a COULTER^{™} counter. This instrument provides a size distribution profile and statistical analysis of the particles. Alternatively, the average size of the particles can be determined by visualization under a microscope fitted with a sizing slide or eyepiece.

If desired, the nucleic acid can be extracted from the microparticles for analysis by the following procedure. Microparticles are dissolved in an organic solvent such as chloroform or methylene chloride in the presence of an aqueous solution. The polymer stays in the organic phase, while the DNA goes to the aqueous phase. The interface between the phases can be made more distinct by centrifugation. Isolation of the aqueous phase allows recovery of the nucleic acid. The nucleic acid is retrieved from the aqueous phase by precipitation with salt and ethanol in accordance with standard methods. To test for degradation, the extracted nucleic acid can be analyzed by HPLC or gel electrophoresis.

### Intracellular Delivery of Microparticles

Microparticles containing DNA are resuspended in saline, buffered salt solution, tissue culture medium, or other physiologically acceptable carrier. For in *vitro*/*ex vivo* use, the suspension of microparticles can be added either to cultured adherent mammalian cells or to a cell suspension. Following a 1-24 hour period of incubation, those particles not taken up are removed by aspiration or centrifugation over fetal calf serum. The cells can be either analyzed immediately or recultured for future analysis.

Uptake of microparticles containing nucleic acid into the cells can be detected by PCR, or by assaying for expression of the nucleic acid. For example, one could measure transcription of the nucleic acid with a Northern blot, reverse transcriptase PCR, or RNA mapping. Protein expression can be measured with an appropriate antibody-based assay, or with a functional assay tailored to the function of the polypeptide encoded by the nucleic acid. For example, cells expressing a nucleic acid encoding luciferase can be assayed as follows: after lysis in the appropriate buffer (e.g., cell lysis culture reagent, Promega Corp, Madison WI), the lysate is added to a luciferin containing substrate (Promega Corp) and the light output is measured in a luminometer or scintillation counter. Light output is directly proportional to the expression of the luciferase gene.

If the nucleic acid encodes a peptide known to interact with a class I or class II MHC molecule, an antibody specific for that MHC molecule/peptide complex can be used to detect the complex on the cell surface of the cell, using a fluorescence activated cell sorter (FACS). Such antibodies can be made using standard techniques (Murphy et al. Nature, Vol. 338, 1989, pp. 765-767). Following incubation with microparticles containing a nucleic acid encoding the peptide, cells are incubated for 10-120 minutes with the specific antibody in tissue culture medium. Excess antibody is removed by washing the cells in the medium. A fluorescently tagged secondary antibody, which binds to the first antibody, is incubated with the cells. These secondary antibodies are often commercially available, or can be prepared using known methods. Excess secondary antibody must be washed off prior to FACS analysis.

One can also assay by looking at T or B effector cells. For example, T cell proliferation, cytotoxic activity, apoptosis, or cytokine secretion can be measured.

Alternatively, one can directly demonstrate intracellular delivery of the particles by using nucleic acids which are fluorescently labeled, and analyzing the cells by FACS or microscopy. Internalization of the fluorescently labeled nucleic acid causes the cell to fluoresce above background levels. Because it is rapid and quantitative, FACS is especially useful for optimization of the conditions for *in vitro* or *in vivo* delivery of nucleic acids. Following such optimization, use of the fluorescent label is discontinued.

If the nucleic acid itself directly affects cellular function, e.g., if it is a ribozyme or an antisense molecule, or is transcribed into one, an appropriate functional assay can be utilized. For example, if the ribozyme or antisense nucleic acid is designed to decrease expression of a particular cellular protein, the expression of that protein can be monitored.

### In Vivo Delivery of Microparticles

Microparticles containing nucleic acid can be injected into mammals intramuscularly, intravenously, intraarterially, intradermally, intraperitoneally, or subcutaneously.

Expression of the nucleic acid is monitored by an appropriate method. For example, expression of a nucleic acid encoding an immunogenic protein of interest is assayed by looking for an antibody or T cell response to the protein.

Antibody responses can be measured by testing serum in an ELISA assay. In this assay, the protein of interest is coated onto a 96 well plate and serial dilutions of serum from the test subject are pipetted into each well. A secondary, enzyme-linked antibody, such as anti-human, horseradish peroxidase-linked antibody, is then added to the wells. If antibodies to the protein of interest are present in the test subject's serum, they will bind to the protein fixed on the plate, and will in turn be bound by the secondary antibody. A substrate for the enzyme is added to the mixture and a colorimetric change is quantitated in an ELISA plate reader. A positive serum response indicates that the immunogenic protein encoded by the microparticle's DNA was expressed in the test subject, and stimulated an antibody response. Alternatively, an ELISA spot assay can be employed.

T cell proliferation in response to a protein following intracellular delivery of microparticles containing nucleic acid encoding the protein is measured by assaying the T cells present in the spleen, lymph nodes, or peripheral blood lymphocytes of a test animal. The T cells obtained from such a source are incubated with syngeneic APCs in the presence of the protein or peptide of interest. Proliferation of T cells is monitored by uptake of ³H-thymidine, according to standard methods. The amount of radioactivity incorporated into the cells is directly related to the intensity of the proliferative response induced in the test subject by expression of the microparticle-delivered nucleic acid. A positive response indicates that the microparticle containing DNA encoding the protein or peptide was taken up and expressed by APCs *in vivo.*

The generation of cytotoxic T cells can be demonstrated in a standard ⁵¹Cr release assay. In these assays, spleen cells or peripheral blood lymphocytes obtained from the test subject are cultured in the presence of syngeneic APCs and either the protein of interest or an epitope derived from this protein. After a period of 4-6 days, the effector cytotoxic T cells are mixed with ⁵¹Cr-labeled target cells expressing an epitope derived from the protein of interest. If the test subject raised a cytotoxic T cell response to the protein or peptide encoded by the nucleic acid contained within the microparticle, the cytotoxic T cells will lyse the targets. Lysed targets will release the radioactive ⁵¹Cr into the medium. Aliquots of the medium are assayed for radioactivity in a scintillation counter. Assays, such as ELISA or FACS, can also be used to measure cytokine profiles of responding T cells.

### Lipid-Containing Microparticles

The microparticles described herein can also include one or more types of lipids. The inclusion of a lipid in a microparticle can increase the stability of the nucleic acid in the microparticle, e.g., by maintaining a covalently closed double-stranded DNA molecule in a supercoiled state. In addition, the presence of a lipid in the particle is believed to modulate, i.e., increase or decrease, the rate at which a drug or nucleic acid is released from the microparticle.

Addition of a lipid to the microparticle can in certain cases increase the efficiency of encapsulation of the nucleic acid or increase the loading of the nucleic acid within microparticles. For example, the encapsulation efficiency may be improved because the presence of the lipid reduces the surface tension between the inner aqueous phase and the organic phase. Reduction of the surface tension is thought to create an environment more favorable for the nucleic acid, and therefore to increase its retention within the microsphere. A reduction in surface tension also allows for the primary emulsion to be formed with less manipulation, which minimizes shearing of the nucleic acid and increases encapsulation efficiency. It is also possible that the presence of lipid in the microparticle may enhance the stability of the microsphere/nucleic acid formulation, and may increase the hydrophobic nature of the microparticles, thereby increasing uptake by phagocytic cells.

The lipids can be cationic, anionic, or zwitterionic, or may carry no charged groups, such as nonpolar glycerides. The lipids preferably are not present as liposomes that encapsulate (i.e., surround) the microparticles. The lipids may optionally form micelles.

Examples of lipids that can be used in the microparticles include acids (such as carboxylic acids), bases (such as amines), phosphatidylethanolamine, phosphatidyl glycerol, phosphatidyl serine, phosphatidyl inositol, phosphatidylcholine, phosphatidic acid, containing one or more of the following groups: propinoyl (trianoic), butyroyl (tetranoic), valeroyl (pentanoic), caproyl (hexanoic), heptanoyl (heptanoic), caproyl (decanoic), undecanoyl (undecanoic), lauroyl (dodecanoic) tridecanoyl (tridecanoic), myristoyl (tetradecanoic), pentadecanoyl (pentadecanoic), palmitoyl (hexadecanoic), phytanoyl (3,7,11,15-tetramethylhexadecanoic), heptadecanoyl (heptadecanoic), stearoyl (octadecanoic), bromostearoyl

(dibromostearoic), nonadecanoyl (nonadecanoic), arachidoyl (eicosanoic), heneicosanoyl (heneicosanoic), behenoyl (docosanoic), tricosanoyl (tricosanoic), lignoceroyl (tetracosanoic), myristoleoyl (9-*cis-*tetradecanoic), myristelaidoyl (9-trans-tetradecanoic), palmitoleoyl (9-cis-hexadecanoic), palmitelaidoyl (9-*trans-*hexadecenoic), petroselinoyl (6-cis-octadecenoic), oleoyl (9-cis- octadecenoic), elaidoyl (9-trans-octadecenoic), linoleoyl (9-cis-12-cis-octadecadienoic), linolenoyl (9-cis-12-cis-15-cis octadecadoenoic), eicosenoyl (11-*cis-*eicosenoic), arachidonoyl (5,8,11,14 (all *cis*) eicosatetraenoic), erucoyl (13-cis-docsenoic), and nervonoyl (15-*cis-*tetraosenoic).

Other suitable lipids include cetyltrimethyl ammonium, which is available as cetyltrimethyl ammonium bromide ("CTAB").

More than one lipid can be used to make a lipid-containing microparticle. Suitable commercially available lipid preparations include lecithin, OVOTHIN 160^{™}, and EPIKURON 135F^{™} lipid suspensions, all of which are available from Lucas Meyer, Inc., Decatur, IL.

The lipid may also be isolated from an organism, e.g., a mycobacterium. The lipid is preferably a CD1-restricted lipid, such as the lipids described in Pamer, Trend Microbiol. 7:13, 1999; Braud, Curr Opin. Immunol. 11:100, 1999; Jackman, Crit. Rev. Immunol. 19:49, 1999; and Prigozy, Trends Microbiol. 6:454, 1998.

In addition to the lipids incorporated into the microparticles, the microparticles can be suspended in a lipid (or lipid suspension) to improve delivery, e.g., by injection.

The relative increase or decrease in release observed will depend in part on the type of lipid or lipids used in the microparticle. Examples of lipids that increase the release of nucleic acid from microparticles include CTAB and the lecithin and OVOTHIN^{™} lipid preparations.

The chemical nature of the lipid can affect its spatial relationship with the nucleic acid in the particle. If the lipid is cationic, it will likely interact directly with the nucleic acid. If the lipid is not charged, it will be interspersed within the microparticle.

The lipid-containing microparticles may also include the stabilizers described above. The inclusion of a lipid in a microparticle along with a stabilizer such as sucrose can provide a synergistic increase in the release of nucleic acids within the microparticle.

Lipid-containing microparticles can be prepared by adding a lipid to either the organic solvent containing the polymer, to the aqueous solution containing the DNA solution, or to the third solution used to make the second emulsion, as described above. The solubility properties of a particular lipid in an organic or aqueous solvent will determine which solvent is used.

Some lipids or lipid suspensions can be added to either the organic solvent or aqueous solution. However, the release properties of the resulting microparticles can differ. For example, microparticles prepared by adding a lecithin lipid suspension to the aqueous nucleic acid-containing solution release amounts similar to or less than the amount released by microparticles prepared without lipids. In contrast, addition of the lecithin lipid suspension to the organic solvent produces microparticles which release more nucleic acid.

Microparticles may in addition be resuspended in a lipid-containing solution to facilitate resuspension and dispersion of the microparticles.

In addition to the lipid-containing microparticles described herein, microparticles may also be made using other macromolecules such as chitin, gelatin, or alginate, or various combinations of these macromolecules and lipids. These microparticles made with these other macromolecules may in addition include the above-described stabilizing agents.

### EXPERIMENT 1: Incorporation of DNA: Analysis of Particle Size and DNA Integrity

### Preparation of DNA for Incorporation

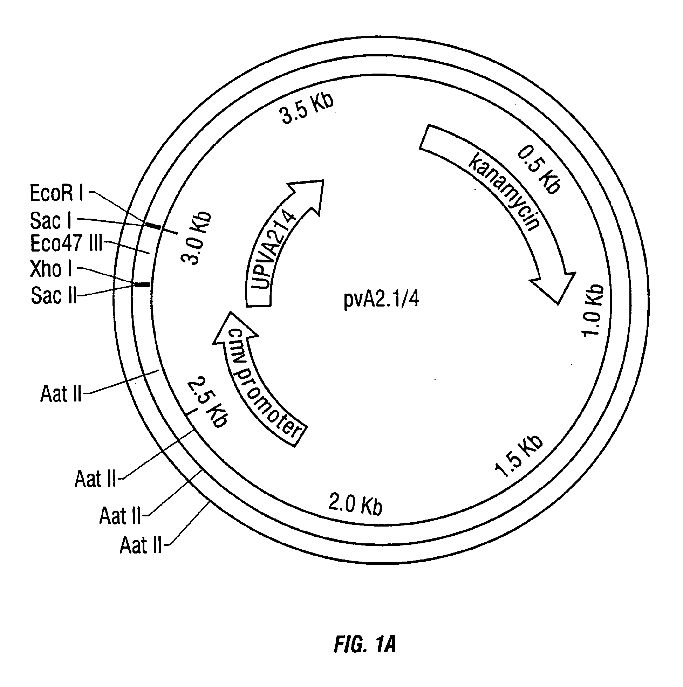

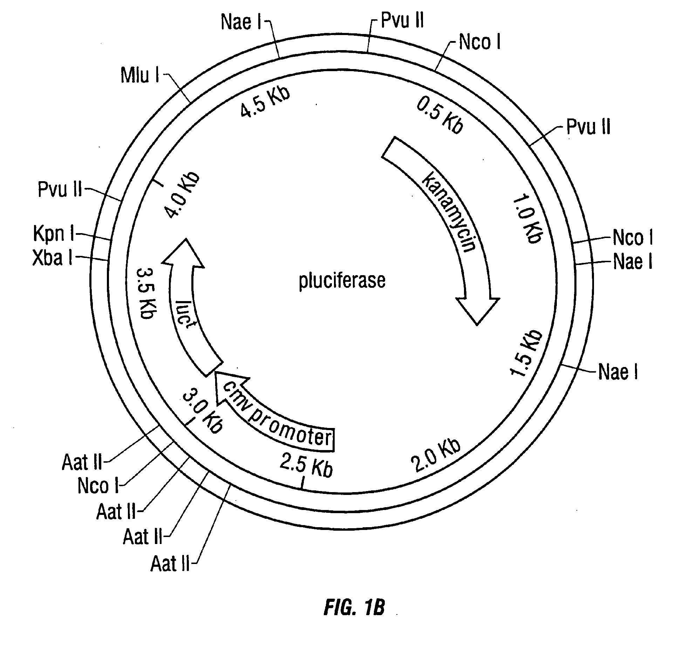

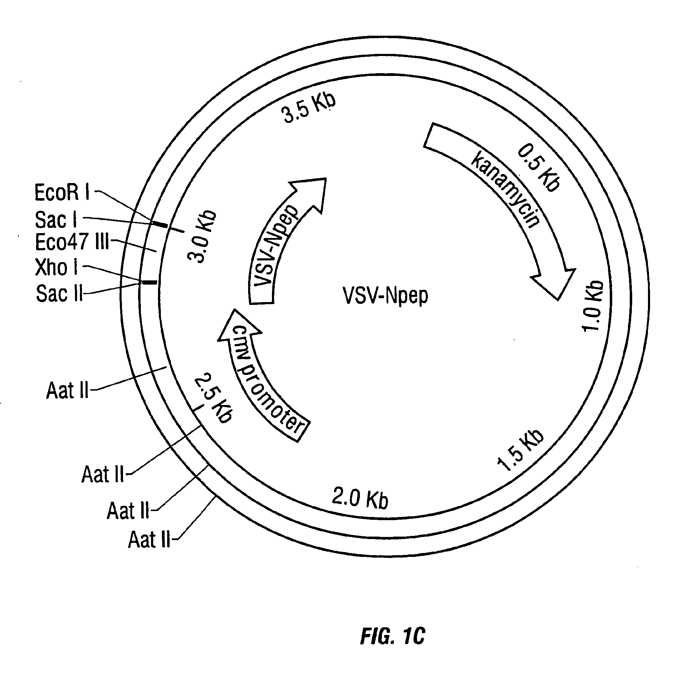

Plasmid DNA was prepared by standard methods using MEGA-PREP^{™} Kit (Qiagen) according to the manufacturer's instructions. An endotoxin-free buffer kit (Qiagen) was used for all DNA manipulations. The DNA was resuspended in distilled, deionized, sterile water to give a final concentration of 3 µg/µl. Fig. 1 shows plasmid maps of DNA expression vectors encoding a) luciferase, b) a vesicular stomatitis virus (VSV) peptide epitope termed VSV-Npep, and c) a human papilloma virus (HPV) peptide epitope termed A2.1/4.

### Association of DNA with PLGA

200 mg of poly-lactic-co-glycolic acid (PLGA) (Aldrich, 65:35 ratio of lactic acid to glycolic acid) was dissolved in 5-7 ml of methylene chloride. 300 µl of the DNA solution prepared above, containing 900 µg DNA, was added to the PLGA solution. The mixture was sonicated in a Model 550 SONIC DISMEMBRATOR^{™} (Fisher Scientific) on setting #3 for 5-60 seconds, and the resulting emulsion was analyzed. An emulsion verified to contain particles of desired size having DNA of satisfactory integrity (as determined below) was added to a beaker containing 50 ml aqueous 1% w/v polyvinyl alcohol (PVA) (mw range: 30-70 kdal). The mixture was homogenized in a POWERGEN^{™} homogenizer (Fisher Scientific) set at 3000-9000 RPM for 5-60 seconds. Again, the DNA integrity was analyzed. In the cases where the DNA was found to be sufficiently intact, the resulting second emulsion was transferred into a second beaker containing 100 ml aqueous 0.05% PVA, with constant stirring. The stirring was continued for 2-3 hours.

The microparticle solution was poured into a 250 ml centrifuge tube and spun at 2000 rpm for 10 minutes. The contents of the tubes were decanted and the sedimented particles were resuspended in 100 ml deionized water. After repeating the centrifugation and decanting steps, the particles were frozen in liquid nitrogen and finally lyophilized until dry.

### Analysis of Microparticle Size Profile

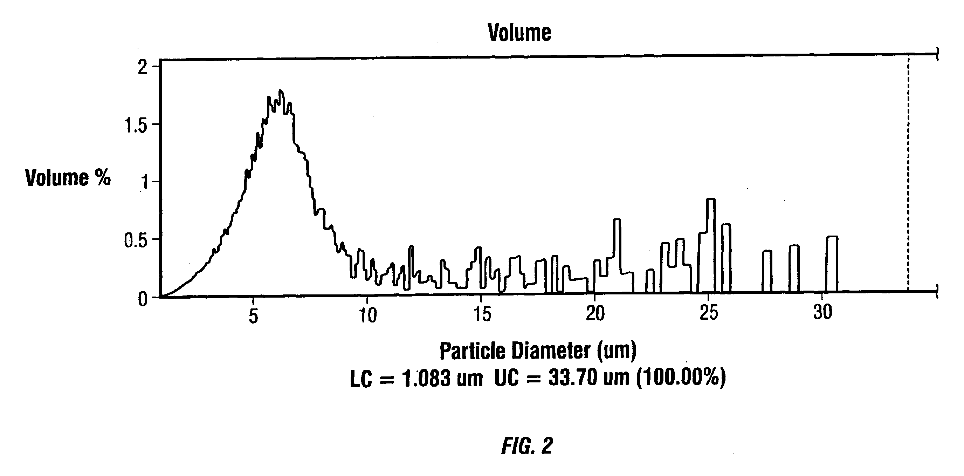

5 mg of the lyophilized microparticles were resuspended in 200 µl water. The resulting suspension was diluted to about 1:10,000 for analysis with a COULTER^{™} counter. Fig. 2 is a print-out from the COULTER^{™} counter which indicates that approximately 85% of the microparticles were between 1.1 and 10 µm in diameter.

### Determination of DNA Integrity

2-5 µg of the microparticles were wet with 10 µl water in an EPPENDORF^{™} tube. 500 µl chloroform was added with thorough mixing to dissolve the polymeric matrix. 500 µl water was added, again with mixing. The resulting emulsion was centrifuged at 14,000 rpm for 5 minutes. The aqueous layer was transferred to a clean EPPENDORF^{™} tube, along with 2 volume equivalents of ethanol and 0.1 volume equivalents of 3M aqueous sodium acetate. The mixture was centrifuged at 14,000 rpm for 10 minutes. After aspiration of the supernatant, the pelleted DNA was resuspended in 50 µl water. DNA was electrophoresed on a 0.8% agarose gel next to a standard containing the input DNA. The DNA on the gel was visualized on a UV light box. Comparison with the standard gives an indication of the integrity of the microparticles' DNA. The microparticle formation procedure was deemed successful if the incorporated DNA retained a high percentage of supercoiled DNA relative to the input DNA.

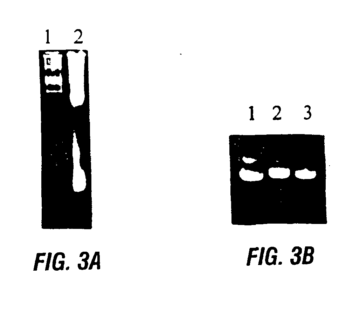

As indicated in Figs. 3A and 3B, homogenization speed and duration are inversely related to DNA integrity. Fig. 3A depicts the DNA isolated from microparticles prepared by homogenization at 7000 rpm for 1 minute (lane 1), and supercoiled input DNA (lane 2). Fig. 3B shows DNA isolated from microparticles prepared by homogenization at 7000 rpm for 5 seconds (lane 1), DNA isolated from microparticles prepared by homogenization at 5000 rpm for 1 minute (lane 2), and supercoiled input DNA (lane 3).

### EXPERIMENT 2 : Preparation of DNA and Microspheres

### DNA preparation

500 ml bacterial cultures were poured into one liter centrifuge bottles. The cultures were centrifuged at 4000 rpm at 20°C for 20 minutes. The media were poured off from the pelleted bacteria. The bacterial pellet was completely resuspended in 50 ml buffer P1 (50mM Tris-Hcl, Ph 8.0; 10mM EDTA; 100 µg/ml RNAse), leaving no clumps. 50 ml of buffer P2 (200 Mm NaOH, 1% SDS) was added with gentle swirling, and the suspensions were incubated at room temperature for five minutes. 50 ml of buffer P3 (3.0 M potassium acetate, Ph 5.5, chilled to 4°C) was added with immediate, gentle mixing. The suspensions were incubated on ice for 30 minutes, then centrifuged at 4000 rpm at 4°C for 30 minutes.

A folded, round filter was wetted with water. When the centrifugation was complete, the supernatant was immediately poured through the filter. The filtered supernatant was collected in a clean 250 ml centrifuge bottle.

15 ml of Qiagen ER buffer was added to the filtered lysate, mixing by inverting the bottle 10 times. The lysate was incubated on ice for 30 minutes.

A Qiagen-tip 2500 column was equilibrated by applying 35 ml QBT buffer (750 Mm sodium chloride; 50 Mm MOPS, Ph 7.0; 15% isopropanol; and 0.15% triton X-100). The column was allowed to empty by gravity flow. The incubated lysate was applied to the column and allowed to enter by gravity flow. The column was washed with 4 x 50 ml Qiagen Endofree QC buffer (1.0 M NaCl; 50 Mm MOPS, Ph 7.0; 15% isopropanol). The DNA was eluted from the column with 35 ml of QN buffer (1.6 M NaCl,; 50 Mm MOPS, Ph 7.0; 15% isopropanol) into a 50 ml polypropylene screwcap centrifuge tube. The DNA suspension was split into two tubes by pouring approximately 17.5 ml of the suspension into a second 50 ml screwcap tube.

Using a sterile 10 ml pipet, 12.25 ml isopropanol was added to each tube. The tubes were closed tightly and thoroughly mixed. The contents of each tube were poured into 30 ml Corex (VWR) centrifuge tubes. Each Corex tube was covered with PARAFILM^{®}. The tubes were centrifuged at 11,000 rpm at 4°C for 30 minutes.

The supernatant was aspirated from each tube and the pellet was washed with 2 ml 70% ethanol. The ethanol was aspirated off. The pellet was air dried for 10 minutes, then resuspended in 0.5-1.0 ml water, and transferred to a sterile 1.5 ml microfuge tube. Preparation of microspheres

200 mg PLGA was dissolved in 7 ml methylene chloride in a 14 ml culture tube. A Fisher Scientific PowerGen 700 homogenizer equipped with a 7 mm mixing head was set to setting 6 and the speed 4.5. A Fisher Scientific Sonic Dismembrator 550 sonicator was set to setting 3.

1.2 mg of DNA in 300 µl H₂O was added to the PLGA solution and the resulting mixture was sonicated for 15 seconds. 50 ml of 1.0% PVA was poured into a 100 ml beaker and placed under the homogenizer. The homogenizer probe was immersed until it was about 4 mm from the bottom of the beaker and the homogenizer was supplied with power. The DNA/PLGA mixture was immediately poured into the beaker and the resultant emulsion was homogenized for 10 seconds. The homogenate was poured into the beaker containing 0.05% PVA.

The resulting emulsion was stirred for two hours, poured into a 250 ml conical centrifuge, and spun at 2000 rpm for 10 minutes. The pelleted microspheres were washed with 50 ml water, transferred to a 50 ml polypropylene centrifuge tube, and spun at 2000 rpm for 10 minutes. The pellet was washed with another 50 ml water and spun again at 2000 rpm for 10 minutes. The pellet was frozen in liquid nitrogen, then lyophilized overnight.

### Extraction of DNA from microspheres for gel analysis

1 ml of microspheres suspended in liquid were removed to a 1.5 ml microfuge tube and spun at 14,000 rpm for 5 minutes. Most of the supernatant was removed. 50 µl of TE buffer (10 Mm Tris-Hcl, Ph 8.0; 1 Mm EDTA) was added and the microspheres were resuspended by flicking the side of the tube.

To isolate DNA from freeze-dried or vacuum-dried microspheres, 2-4 mg microspheres were weighed out into a 1.5 ml microfuge tube. 70 µl TE buffer was added, and the microspheres were resuspended.

200 µl chloroform was added to each tube and the tubes were vigorously, but not violently, shaken for two minutes to mix the aqueous and organic layers. The tubes were centrifuged at 14,000 rpm for 5 minutes. 30 µl of the aqueous phase was carefully removed to a new tube.

### PicoGreen and Gel Analysis of Microspheres

3.5-4.5 mg microspheres were weighed out into a 1.5 ml microfuge tube. 100 µl DMSO was added to each tube, and the tubes were rotated at room temperature for 10 min. The samples were removed from the rotator and visually inspected to verify that the samples were completely dissolved. Where necessary, a pipet tip was used to break up any remaining clumps. None of the samples were allowed to remain in DMSO for more than 30 minutes.

For each sample to be tested, 990 µl TE was pipetted into three separate microfuge tubes. 10 µl of the DMSO/microsphere solution was pipetted into each 990 µl TE with mixing. The mixtures were centrifuged at 14,000 rpm for 5 minutes.

For each sample, 1.2 ml TE was aliquoted into a 5 ml round bottom snap cap centrifuge tube. 50 µl of the 1 ml TE/DMSO/microsphere mixture to the 1.2 ml TE. 1.25 ml of PicoGreen (Molecular Probes, Eugene, OR) reagent was added to each tube, and the fluorescence was measured in a fluorimeter.

### EXPERIMENT 3: Alcohol Precipitation

### Ethanol precipitation

DNA was prepared as in EXPERIMENT 2. Three samples, each containing 1.2 mg DNA, were precipitated by the addition of 0.1 vol 3 M sodium acetate and 2 volumes of ethanol. The DNA was resuspended in water to a final concentration of 4 mg/ml. DNA in two of the samples was resuspended immediately before use, and DNA in the third sample was resuspended and then rotated for 4 hours at ambient temperature. Control DNA at 4mg/ml was not ethanol precipitated.

Each of the four samples was encapsulated into microspheres by the procedure described in Experiment 2. The amount of DNA per mg of microspheres was determined by PicoGreen analysis, as described in Experiment 2. The following results were obtained:

| Sample | mg of MS | µg DNA/mg MS | % incorp. | % incr. |
|---|---|---|---|---|
| Ethanol, 0 hr #1 | 4.66 | 3.37 | 56 | 44 |
| Ethanol, 0 hr #2 | 4.45 | 4.91 | 82 | 62 |
| Ethanol, 4 hr | 3.96 | 4.30 | 72 | 57 |
| Unprecip. | 3.97 | 1.85 | 31 | - |

The results indicate that ethanol precipitation of DNA prior to encapsulation in microspheres resulted in increased incorporation ranging from 31% to greater than 56%, representing a 44-62% increase in the amount of encapsulated DNA.

The following experiments verify that the ethanol-precipitation effects observed above are independent of DNA preparation procedures.

DNA was prepared at three different facilities. Sample #1 was prepared as in Experiment 2. Sample #2 was prepared as in Experiment 2, but without the addition of ER-removal buffer. Sample #3 was prepared in a scaled-up fermentation manufacturing run. The three DNA samples were representative of two different plasmids (DNA-1 and DNA-3 were identical) of sizes 4.5 kb and 10 kb. The three DNA samples were tested for the enhancement of encapsulation efficiency by ethanol precipitation. Three samples of DNA, each containing 1.2 mg, were precipitated by the addition of 0.1 vol 3 M sodium acetate and 2 volumes ethanol. The DNA was resuspended in water at a concentration of 4 mg/ml. Three control DNA samples, at 4mg/ml, were not ethanol precipitated.

Each of the samples was encapsulated by the procedure described in Experiment 2.

The amount of DNA per mg of microspheres was determined by PicoGreen analysis as described in Experiment 2. The following results were obtained:

| Sample | mg of MS | µg DNA/mg MS | % incorp. | % incr. |
|---|---|---|---|---|
| #1 eth. ppt. | 3.35 | 3.10 | 67 | 59 |
| #2 eth. ppt. | 4.45 | 4.91 | 66 | 47 |
| #3 eth. ppt. | 3.34 | 2.65 | 48 | 29 |
| #1 unppt. | 3.38 | 1.95 | 42 | - |
| #2 unppt. | 3.35 | 1.80 | 45 | - |
| #3 unppt. | 3.33 | 1.81 | 37 | - |

The data show that ethanol precipitation increased the amount of DNA encapsulated in microspheres by 29-59%. The effect was demonstrated to hold regardless of size and preparation technique.

### Isopropanol vs. ethanol precipitation

Plasmid DNA was precipitated with ethanol or isopropanol, then resuspended in water for 4 hours or 16 hours. Control DNA was not precipitated. Microspheres were made according to the protocol in Experiment 2. The following results were obtained:

| Sample | mg of MS | µg DNA/mg MS | % incorp. | % incr. |
|---|---|---|---|---|
| unppt. #1 | 4.43 | 0.99 | 17 | - |
| unppt. #2 | 4.30 | 0.99 | 17 | - |
| eth. ppt. #1 16 hr | 4.26 | 2.12 | 37 | 118 |
| eth. ppt. #2 16 hr | 4.34 | 1.66 | 31 | 82 |
| isopro. ppt. #1 16 hr | 4.60 | 1.71 | 31 | 82 |
| isopro. ppt. #2 16 hr | 4.90 | 1.72 | 32 | 88 |
| eth. ppt. #1 4 hr | 4.65 | 2.22 | 42 | 147 |
| eth. ppt. #2 4 hr | 4.27 | 1.69 | 30 | 76 |
| isopro. ppt. #1 4 hr | 4.55 | 1.41 | 25 | 47 |
| isopro. ppt. #2 4 hr | 4.30 | 2.78 | 46 | 170 |

These data demonstrate that alcohol precipitation increased the encapsulation efficiency of DNA, independent of the type of alcohol used to precipitate DNA and independent of the time following DNA precipitation.

### Conductivity

The conductivities of the ethanol-precipitated and non-precipitated DNA samples were determined using a conductivity meter. It was found that precipitation of the DNA led to a decrease in the amount of salt present. The conductivity without ethanol precipitation was 384 µΩ, while the conductivity after ethanol precipitation was 182 µΩ. Thus, alcohol precipitation, or any other means of salt/contaminant removal is likely to increase encapsulation efficiency. It therefore appears that treatments that render DNA free from contaminants are likely to increase the efficiency of DNA encapsulation.

DNA was then ethanol precipitated or precipitated in the presence of 0.4M NaCl and 5% hexadecyltrimethylammonium bromide (CTAB). The DNA was then encapsulated as described above. The DNA was extracted and analyzed by agarose gel electrophoresis. The results indicated that precipitation of the DNA with CTAB led to a marked increase in the amount of supercoiled DNA within the microspheres. However, this was accompanied by a decrease in the encapsulation efficiency (6%, rather than 26%).

### EXPERIMENT 4 : Addition of Stabilizer Compounds

### TE buffer

Plasmid DNA was resuspended in TE buffer following ethanol-precipitation, in an attempt to increase DNA stability. The microspheres were then prepared as described in Experiment 2. DNA was extracted from the microspheres and analyzed by agarose gel electrophoresis. One lane was loaded with the input plasmid (pIiPLPLR); another lane with the plasmid DNA following ethanol precipitation, resuspension in water, and encapsulation in microspheres; and still another lane with the plasmid DNA following ethanol precipitation, resuspension in TE buffer, and encapsulation in microspheres. The results indicated that the amount of supercoiled DNA within microspheres was increased by resuspension in TE buffer.

Two other plasmids, designated pbkcmv-n-p and E3PLPLR, were subjected to the conditions described above. This experiment confirmed that the two other plasmids were also stabilized by the TE buffer.

The following experiment was conducted to determine the timing of the TE effect. 2 g PLGA was dissolved in 18 ml methylene chloride. 500 µg DNA was ethanol-precipitated and dissolved in 3.6 ml TE or water. The two solutions were mixed by inverting several times and then sonicated in the Fisher apparatus (see Example 2) on setting 3 for 10 seconds with a 1/8" microtip. At various times after sonication (i.e., 5, 15, 30, 45, and 60 minutes), a 1 ml sample was removed from each tube, 100 µl water was added, the sample was centrifuged in an Eppendorf centrifuge, and the top layer of the centrifugated sample removed to a separate tube. The samples were then analyzed by gel electrophoresis.

The results indicated that TE buffer acted to stabilize the DNA early in the encapsulation process, during formation of the oil in water emulsion.

To determine the effect of Tris and/or EDTA in the TE buffer, DNA was resuspended in water, TE buffer, 10 Mm TRIS, or 1 Mm EDTA prior to encapsulation in microspheres by the method of Experiment The DNA was extracted from the microspheres and analyzed on an agarose gel. Tris and EDTA were each found to be similar to the complete TE buffer in their ability to protect DNA during the encapsulation process and during lyophilization.

An experiment was carried out to determine the effect of Ph on encapsulation (the Ph of the EDTA, Tris, and TE solutions in the previous experiment were all similar). Microspheres were made by encapsulating DNA that had been ethanol precipitated and resuspended in Tris of different Ph, or in phosphate buffered saline (PBS). The DNA was extracted after lyophilization of the particles, and analyzed on agarose gel. The results indicated that there was a significant Ph effect on the stability of encapsulated DNA. Resuspension of the DNA in water (Ph 6.5), PBS (Ph 7.3), and Tris (Ph 6.8) all led to a decrease in the ratio of supercoiled DNA relative to total DNA within the microspheres. Increasing the Ph to 7.5 or higher had a positive effect on the amount of supercoiling, suggesting that basic Ph levels are important for maintaining DNA stability. Increased Ph also had an effect on encapsulation efficiency:

| **SAMPLE** | mg of MS | µg DNA/mg MS | % incorp. |
|---|---|---|---|
| Tris Ph 6.8 | 2.42 | 2.77 | 55.5 |
| Tris Ph 7.5 | 2.52 | 2.73 | 54.6 |
| Tris Ph 8.0 | 2.49 | 3.29 | 65.9 |
| Tris Ph 9.9 | 2.46 | 3.81 | 76.3 |
| water | 2.46 | 2.48 | 49.7 |
| PBS Ph 7.3 | 2.49 | 0.55 | 11 |
| TE Ph 8.0 | 2.52 | 2.22 | 44.3 |

### Other buffer compounds

Borate and phosphate buffers were also tested for their effect on the quality of encapsulated DNA. DNA was ethanol precipitated, resuspended in various buffer solutions, and encapsulated according to the procedure of Experiment 2. The DNA was extracted from the microspheres and analyzed by agarose gel electrophoresis. TE, BE, and PE all afforded greater than 50% supercoiling in the encapsulated DNA. An added benefit to DNA was also discovered, resulting from EDTA in the presence of Tris, borate, or phosphate.

### Other stabilizer compounds

In addition to buffers, other compounds were tested for their ability to protect the DNA during the encapsulation procedure. Plasmid DNA was ethanol-precipitated and resuspended in water or a solution of dextran sulfate. Microspheres were then prepared according to the method of Experiment 2. DNA was extracted from the microspheres before and after lyophilization and analyzed by agarose gel electrophoresis.

The results suggested that the addition of a stabilizer led to encapsulation of more supercoiled DNA than did resuspension of DNA in water alone. The greatest improvement in DNA structure was observed with a 10% dextran sulfate solution. Protection apparently occurred at two levels. An effect of dextran sulfate was seen on DNA pre-lyophilization, as, following encapsulation, a greater proportion of DNA remained in the supercoiled state with increasing amounts of dextran sulfate. The protection rendered by the stabilizer also occurred during the lyophilization procedure, since the presence of the stabilizer during this process increased the percentage of DNA remaining in the supercoiled state.

To determine whether or not the effects of TE and other stabilizers were additive, ethanol-precipitated DNA was resuspended in TE or water, with or without a solution of another stabilizer (e.g., sucrose, dextrose, or dextran). Microspheres were prepared according to the method of Experiment 2. DNA was extracted from the microspheres and analyzed by agarose gel electrophoresis.

The results demonstrated that resuspending DNA in a stabilizer/TE solution is slightly better or equivalent to the use of TE alone, insofar as a greater percentage of DNA remains in the supercoiled state after encapsulation under these conditions.

Stabilizers were also added in combination, to determine whether or not the stabilizer effects are additive. DNA was ethanol-precipitated and resuspended in various stabilizer solutions. The DNA was encapsulated as described in Experiment 2, extracted, and analyzed by agarose gel electrophoresis. The results indicated that combinations of stabilizers can be used to increase the amount of encapsulated, supercoiled DNA.

### EXPERIMENT 5: Addition of Excipients

To determine whether or not excipient compounds have an adverse effect on encapsulated plasmid DNA, microspheres were prepared from ethanol-precipitated DNA following the protocol in Experiment 2, with the exception that prior to lyophilization, the microspheres were resuspended in solutions containing excipients. Each sample was then frozen and lyophilized as in Experiment 2. The final concentration of the excipients in the microspheres upon resuspension at 50 mg/ml was 0.1% Tween 80, 5% D-sorbitol, 5% D-mannitol, or 0.5% carboxymethylcellulose (CMC). DNA was extracted from the microspheres and analyzed on an agarose gel.

The results illustrated that addition of excipients prior to lyophilization did not significantly affect DNA stability or the degree of supercoiling.

### EXPERIMENT 6: In Vitro Cell Studies

### In vitro Phagocytosis of DNA-Containing Microparticles

Into each of two wells of a six-well tissue culture dish, about 10⁶ macrophages were plated in 3 ml RPMI medium containing 10% fetal calf serum. 5 mg of the microparticles containing DNA encoding luciferase were resuspended in 200 µl saline solution, and 50 µl of the resulting suspension was added to one of the wells containing macrophages. The plate was incubated at 37°C for 1-6 hours. Side vs. forward scatter (i.e., intracellular complexity vs. size) of the cells was analyzed by FACS using a Becton Dickinson FACS instrument.

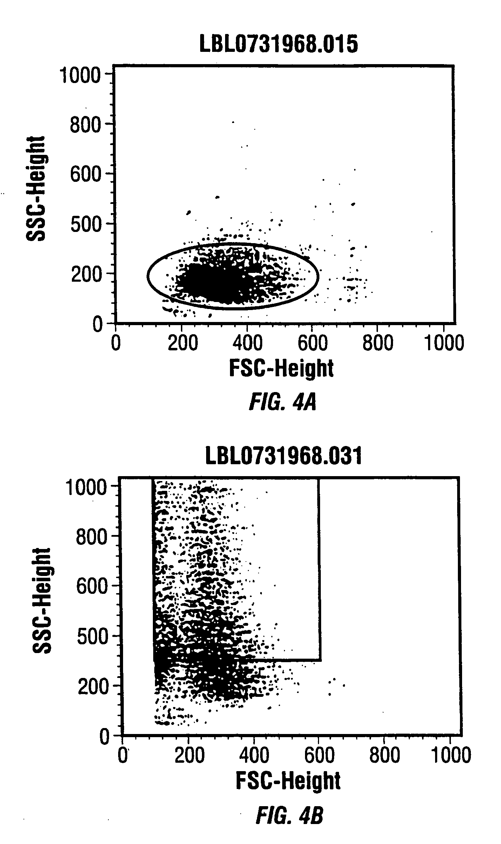

Fig. 4 shows the results. Cell populations that have not phagocytosed are found in region R1. Phagocytosing cells remain the same size (FSC profile), but demonstrate an increased side scatter profile. These cells are found in region R2.

### Measurement of DNA Expression Following Phagocytosis

Into two wells of a 24-well tissue culture dish, about 2.5×10⁵ macrophages were plated in 1 ml RPMI medium containing 10% fetal calf serum. The plate was incubated at 37°C for 6 hours. 1 mg of the lyophilized microparticles containing DNA encoding luciferase was resuspended in 400 µl saline solution. 6 µl of the resulting suspension was added to one of the wells containing macrophages, and 25 µl of suspension was added to the other. The plate was incubated at 37°C for 4 hours. The medium, including the microparticles, was removed and fresh medium added to the cells. The plate was again incubated at 37°C for 1-5 days. The cells were harvested into a tube and spun at 1,500 RPM for 5 minutes. The pelleted cells were resuspended in 100 µl of 1X Cell Lysis Buffer (Promega) in an EPPENDORF™ tube. The mixture was centrifuged at 14,000 RPM for 5 minutes in order to precipitate out any cell debris. The cell lysate was assayed by adding 5 µl of the supernatant to 100 µl of luciferase substrate (Promega) and measuring the light output on a TOPCOUNT™ combination luminometer/scintillation counter (Packard Instruments).

The data for this experiment are provided in Table 5. They indicate that cells phagocytosing microparticles that contain, for example, luciferase DNA, do in fact express the DNA. Thus, DNA integrity and functionality are confirmed. The data also indicate that the uptake of the microparticles by phagocytosis does not prevent the DNA from reaching the nucleus.

**TABLE 5 : Phagocytosis of encapsulated DNA leads to expression of a luciferase reporter gene construct.**

| MICROPARTICLES CONTAINING: | | | | |
|---|---|---|---|---|
| | **Luciferase DNA** | | **Control DNA** | |
| | 25 µl | 6 µl | 25 µl | 6 µl |
| **Day 1** | 1257 | 168 | 103 | 245 |
| **Day 2** | 2632 | 492 | 107 | 133 |
| **Day 3** | 3400 | 507 | 80 | 93 |
| **Day 5** | 763 | 310 | 90 | 90 |

| | | | | |
|---|---|---|---|---|
| Data given in counts per 0.01 minute | | | | |

### EXPERIMENT 7 : In Vivo Cell Studies

### In Vivo Expression of Incorporated DNA

45 mg of luciferase cDNA in microparticles was resuspended in 250 µl saline solution. 40 µl of the resulting suspension was injected into each tibialis anterior muscle of a mouse. Seven days later, each tibialis anterior was dissected and placed in an EPPENDORF™ tube on dry ice. Using a mortar and pestle cooled with dry ice, each tibialis anterior muscle was ground into a powder, then return to the EPPENDORF™ tube. 500 µl 1X cell lysis buffer (Promega) was added. The tube was shaken upside-down on a vortex mixer at 4°C for 15 minutes. The tube and its contents were frozen in liquid nitrogen, then thawed to 37°C. The freeze/thaw cycle was repeated two more times. The tube was centrifuged 14,000 RPM for 10 minutes. The supernatant was transferred to a new tube and centrifuged again for 5 minutes. To assay for expression, 20 µl of the supernatant was added to 100 µl of luciferase substrate (Promega) and the light output was measured on a TOPCOUNT™ combination luminometer/scintillation counter (Packard Instruments).

The data for this experiment are provided in Table 6. They indicate that muscle cells can express DNA released from microparticles. Since these cells are not known to phagocytose, this is an example of depot effect.

**TABLE 6: Expression of encapsulated luciferase DNA in murine muscles**

| | |
|---|---|
| **Muscle 1** | 2 x 10⁵ |
| **Muscle 2** | 8 x 10⁴ |
| **Muscle 3** | 1 x 10⁶ |
| **Muscle 4** | 6 x 10⁵ |
| **Control** | 2 x 10² |

| | |
|---|---|
| Data given in counts per 0.01 minute | |

### Generation of Cytotoxic T Cells Following Injection of Microparticles Containing DNA

90 mg of microparticles containing DNA encoding VSV-Npep was resuspended in 900 µl of saline solution. 60 mg of microparticles containing control vector DNA was resuspended in 600 µl of saline solution. 300 µg VSV-Npep plasmid DNA was resuspended in 300 µl of saline solution. 300 µg control vector DNA was resuspended in 300 µl of saline solution. 150 µg of the VSV-N peptide was resuspended in incomplete Freund's adjuvant (IFA).

The five suspensions were injected intraperitoneally, intramuscularly, or subcutaneously, according to the following regimen:
1. Intraperitoneal : A first group of 3 mice was injected intraperitoneally with 100 µl of microparticles containing VSV-Npep DNA (Group 1). A second group of 3 mice was injected with 100 µl of microparticles containing control vector DNA (Group 2).
2. Intramuscular: (into each tibialis anterior muscle): A third group of 3 mice was injected intramuscularly with 100 µl of microparticles containing VSV-Npep DNA (Group 3). A fourth group of 3 mice was injected with 100 µl microparticles containing control vector DNA (Group 4). A fifth group of 3 mice was injected with 50 µg/leg VSV-Npep plasmid DNA (i.e., in the absence of microparticles) (Group 5). A sixth group of 3 mice was injected with 50 µg/leg control vector plasmid DNA (Group 6).
3. Subcutaneous: A seventh group of 3 mice was injected subcutaneously with 100 µl of microparticles containing VSV-Npep DNA (Group 7). An eighth group of 3 mice was injected with 50 µg VSV-N peptide/IFA (Group 8).

After two weeks, groups 5, 6, and 8, which received either synthetic peptide or DNA without microparticles, were injected again. Groups 1-4 and 7, which initially received microparticles, were not reinjected.

Seven days after the last set of injections, the murine spleens were harvested. Single cell suspensions were generated by standard methods, the red blood cells were lysed, and the remaining cells were resuspended in RPMI with 10% fetal calf serum to give a final concentration of 4×10⁶ effector cells/ml. Half of the cells from each group were then incubated at 37°C for 6 days with an equal number of peptide-pulsed syngeneic stimulator cells which had been previously treated with mitomycin C. The remaining cells were incubated with 50 µM peptide alone.

After the second day of incubation, 0.1 volume equivalents of IL-2-containing supernatant, derived from cells incubated in ConA, was added. After the sixth day of incubation, the effector cells were harvested and incubated in 96-well round-bottom plates containing ⁵¹Cr-labeled, peptide-pulsed target cells at 37°C for 5 hours. The effector-to-target ratios for the wells ranged from 200:1 down to 1:1.

To determine the level of maximal lysis, 20 µl of aqueous 10% sodium dodecyl sulfate (SDS) was added to certain wells containing only target cells. To determine the level of spontaneous lysis, certain wells were incubated with media alone (i.e., target cells but no effector cells). Specific lysis is calculated as follows: [(experimental lysis)-(spontaneous lysis)/(maximal lysis)-(spontaneous lysis)] × 100 = specific lysis.

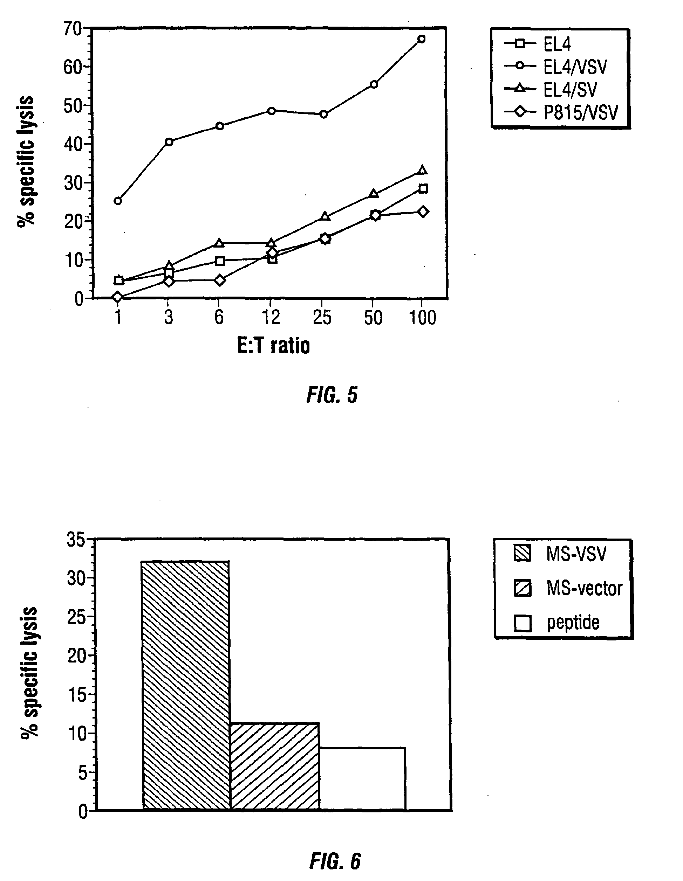

The results are shown in Figs. 5-9.

In the experiment associated with Fig. 5, effector cells from mice (Group 1) immunized intraperitoneally with microparticles containing DNA that encodes a peptide from the VSV-N protein were tested for cytolytic activity against various target cells. The VSV peptide binds to the mouse H-2K^{b} class I receptor. Syngeneic targets express the H-2K^{b} receptor while the allogeneic targets used in this experiment express the H-2K^{d} receptor.

CTL activity was tested on syngeneic targets (EL4) without peptide, syngeneic targets (EL4/VSV) labeled with the VSV peptide, syngeneic targets (EL4/SV) labeled with SV peptide (i.e., a non-specific peptide), and allogenic targets (P815/VSV) labeled with VSV peptide.

Because the allogeneic targets (P815/VSV) do not express the H-2K^{b} receptor, they should not be recognized and lysed by the effector cells. Thus, P815 targets mixed with the VSV peptide are not lysed. Syngeneic targets (EL4) that do not have the VSV peptide are also not lysed. Syngeneic targets (EL4/SV) that express a peptide different from VSV are also not lysed. Only those targets (EL4/VSV) that have both the right MHC receptor and the right peptide are lysed.

Together, the data demonstrate that CTL activity can be elicited by immunization with microparticles containing DNA that encodes a VSV peptide, and the lysis is MHC restricted and peptide specific. In other words, only the right peptide with the right MHC receptor is recognized by the T cell receptor of the CTL generated by immunization in accordance with the invention. This demonstrated that the microparticles serve the desired function.

Next, the CTL response generated by immunizing mice subcutaneously with synthetic peptide (Group 8) was compared with the CTL response generated by immunizing mice intraperitoneally with microparticles containing DNA that encodes the VSV peptide (Groups 1 and 2). In Fig. 6 is shown the lysis obtained at a E:T ratio of 100:1 for CTL generated by immunizing the mice with either microparticles including DNA that encodes the VSV-N peptide (MS-VSV; Group 1), microparticles including control vector DNA that does not encode a VSV peptide (MS-vector; Group 2), or synthetic VSV-N peptide (peptide; Group 8). The targets were syngeneic (EL4) cells labelled with VSV peptide.

Mice immunized with the VSV-Npep DNA in microparticles (MS-VSV) generated a stronger CTL response (33% specific lysis) than mice immunized with control microparticles containing empty vector DNA (MS-vector) (10% specific lysis). Mice immunized with VSV-N peptide (peptide) generate a weaker CTL response than those immunized with microparticles containing VSV-Npep DNA (MS-VSV). Therefore, the microparticles served the desired function.

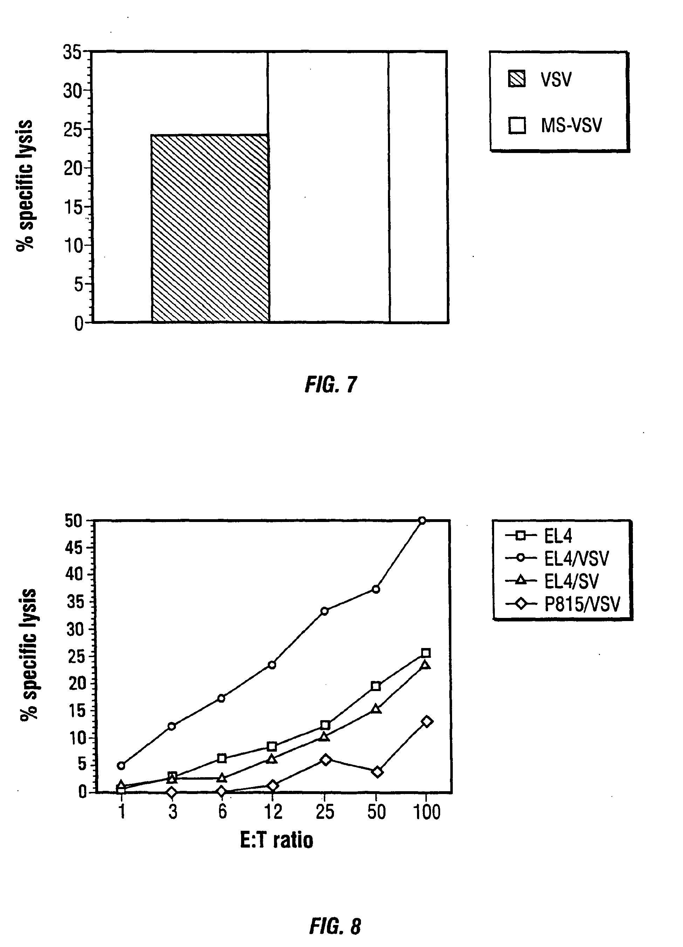

CTL responses in mice immunized intraperitoneally with VSV-Npep DNA contained in microparticles (MS-VSV) were compared with the CTL responses of mice immunized intramuscularly with "naked" VSV DNA (VSV). CTL responses in mice immunized with the microparticles containing DNA (MS-VSV; Group 1) were stronger than those in mice immunized with naked DNA (VSV; Group 5) at an E:T ratio of 3:1 (Fig. 7). The targets were syngeneic (EL4) cells labelled with VSV peptide. The mice which received naked DNA were immunized twice, while the mice immunized with microparticles were only given one treatment. The data in Fig. 7 therefore show that one injection of DNA in microparticles was more effective than two injections of a greater amount of naked DNA.

Fig. 8 shows the results of an experiment equivalent to that related in Fig. 5, with the exception that the injections were subcutaneous (Group 8 mice) instead of intraperitoneal. This experiment demonstrated that subcutaneous injections of microparticles containing VSV-Npep DNA are also effective for producing CTL responses.

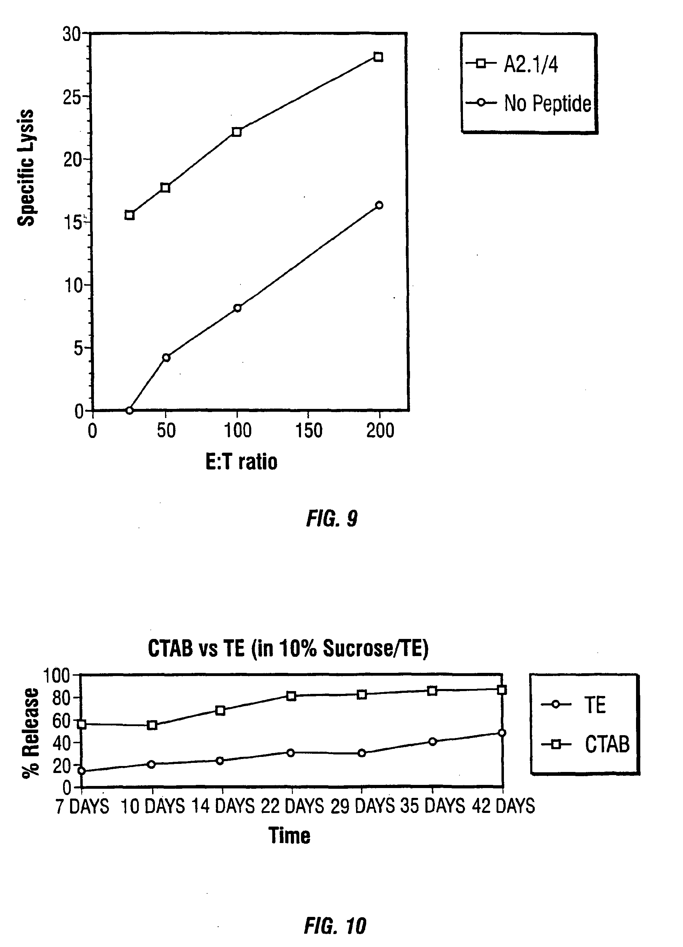

The experiment illustrated in Fig. 9 is also similar to that of Fig. 5, except that DNA encoding a different peptide was used in order to demonstrate that the results obtained were not unique to VSV-Npep DNA. HLA-A2 transgenic mice were immunized with microparticles containing DNA that encodes a peptide from human papilloma virus (HPV) E7 peptide. The HPV E7 peptide termed A2.1/4 binds to the human MHC receptor HLA-A2. The experiment assessed the ability of CTL effectors to lyse syngeneic targets' (i.e., targets having the correct HLA receptor) that were either labeled with the correct HPV peptide (A2.1/4) or else unlabeled (no peptide). The E:T ratios are listed along the X-axis.

### EXPERIMENT 8: Treatment with Microparticles Containing DNA

According to the procedure of Experiment 1, microparticles are prepared containing DNA encoding a peptide having an amino acid sequence about 50% identical to PLP residues 170-191 (SEQ ID NO: 2). A multiple sclerosis patient whose T cells secrete excess T_{H}1 cytokines (i.e., IL-2 and γ-IFN) in response to autoantigens is injected intravenously with 100 µl to 10 ml of the microparticles. Expression of the PLP-like peptide by APCs results in the switching of the cytokine profile of the T cells, such that they instead produce T_{H}2 cytokines (i.e., IL-4 and IL-10) in response to autoantigens.

### EXPERIMENT 9 : Tolerizing with Microparticles Containing DNA

According to the procedure of Experiment 1, microparticles are prepared containing DNA encoding a peptide having an amino acid sequence corresponding to MBP residues 33-52 (SEQ ID NO: 34). A mammal is injected subcutaneously with 1-500 µl of the microparticles. Expression of the MBP peptide by APCs results in the tolerization of T cells that recognize the autoantigen.

### EXPERIMENT 10 : Implantation of Microparticles

A DNA molecule, including an expression control sequence operatively linked to a sequence encoding both a trafficking sequence and a peptide essentially identical to myelin basic protein (MBP) residues 80-102 (SEQ ID NO: 1), is associated with a polymer to form microparticles, according to the procedure of Experiment 1. Particles smaller than 100 µm are removed. The polymeric constituent of the microparticle is poly-lactic-coglycolic acid, where the ratio of lactic acid to glycolic acid is 65:35 by weight. The resulting microparticles are surgically implanted subcutaneously in a patient.

### EXPERIMENT 11 : Preparation of Microparticles Containing Both DNA and Protein

Plasmid DNA is prepared by standard methods using MEGA-PREP™ Kit (Qiagen) according to the manufacturer's instructions. An endotoxin-free buffer kit (Qiagen) is used for all DNA manipulations. The DNA is resuspended in distilled, deionized, sterile water to give a final concentration of 3 µg/µl. Additionally, 0-40 mg of purified protein is added to about 1 ml of the DNA solution. A mass of gelatin, equal to about 20 % of the mass of protein, is added.

Up to 400 mg of PLGA (i.e., at least ten times the mass of protein) is dissolved in about 7 ml methylene chloride. The DNA/protein solution is poured into the PLGA solution and homogenized or sonicated to form a first emulsion. The first emulsion is poured into about 50-100 ml of an aqueous solution of surfactant (e.g., 0.05% to 2% PVA by weight). The mixture is homogenized at about 3000-8000 RPM to form a second emulsion. The microparticles are then isolated according to the procedure of Experiment 1.

### EXPERIMENT 12 : Treatment with Microparticles Containing Both DNA and Protein

Microparticles including both an antigenic protein having the conformational determinants necessary for induction of B cell response against hepatitis B virus (HBV) and DNA encoding the CTL epitope for HBV, are prepared according to the procedure of Experiment 10. A patient infected or at risk of infection with HBV is immunized with the microparticles.

Slow release of the protein from non-phagocytosed microparticles leads to B cell recognition of the conformational determinants and subsequent secretion of antibody. Slow release of the DNA or phagocytosis of other microparticles causes APCs (1) to express the DNA of interest, thereby generating a T cell response; and (2) to digest the protein released from the microparticles, thereby generating peptides which are subsequently presented by class I or II molecules. Presentation by class I molecules promotes CTL response; presentation by class II molecules promotes both antibody and T cell responses, since T_{H} cells activated by the class II/peptide complexes secrete non-specific cytokines.

The results are elimination of HBV from the patient and continued prevention of production of virus within the patient's cells.

### EXPERIMENT 13: Phagocytosis of Microspheres Containing Plasmid DNA by Murine Dendritic Cells

Microspheres were prepared by the procedure of Experiment 2, except that a fluorescent oligonucleotide was added during the encapsulation procedure. Splenic dendritic cells were isolated from mice and incubated with nothing, with fluorescent beads, or with the prepared microspheres. FACS analysis of the cells indicated that the fluorescent beads and the prepared microspheres were both phagocytosed. Moreover, the prepared microspheres did not fluoresce unless they had been ingested by the dendritic cells, suggesting that following phagocytosis, the microparticles became hydrated and degraded, allowing release the encapsulated DNA into the cell cytoplasm.

### EXPERIMENT 14 : Preparation of Lipid-Containing Microparticles

To prepare lipid-containing microparticles, 200 mg PLGA was dissolved in 7 ml of methylene chloride ("DCM") (J.T. Baker, Catalog # 9324-11) in a 14 ml tube. The resulting PLGA/DCM solution was poured into a 35 ml polypropylene cylindrical tube prepared by truncating a 50 ml polypropylene cylindrical tube at the 35 ml mark. An OVOTHIN™ lipid solution was added to the PLGA/DCM solution to a final concentration of 0.05% (vol/vol).

A Silverson SL2T homogenizer (East Longmeadow, MA) with a 5/8 inch slotted mixing head was preset at setting 10. Prior to beginning homogenization, 50 ml of a 1.0% PVA solution (Average MW: 23,000: 88% hydrolyzed) was poured into a 100 ml beaker, and 100 ml of 0.05% PVA/300 Mm sucrose solution was poured into a 250 ml beaker containing a 1.5 inch stir bar. The beaker was placed on a stir plate.

1.2 mg of pBVKCMluc DNA in 300 µl TE/10% SDS was added to the PLGA/DCM solution. The mixture was homogenized for 2 min. at room temperature to form a DNA/PLGA emulsion. The homogenizer was then shut off and the DNA/PLGA emulsion removed. The 1.0% PVA solution (50 Ml) was placed under the homogenizer probe, and homogenization resumed. The DNA/PLGA emulsion was immediately poured into the beaker containing the 1.0% PVA solution, and the mixture homogenized for 1 minute. The mixture was then poured into the beaker containing 0.05% PVA on the stir plate and stirred for two hours.

After two hours, the mixture was poured into a 250 Ml conical centrifuge tube and spun in a Beckman GS6R clinical centrifuge at 2500 rpm for 10 min. The pelleted microparticles were washed twice with water.

After the second washing the pellet was resuspended in water, frozen in liquid nitrogen and lyophilized for at least 11 hours.

DNA from microparticles prepared using TE/sucrose was present in a concentration of 2.33µg/ml (DNA/PLGA) and 55% supercoiling, whereas DNA from microparticles prepared using OVOTHIN™ lipid was present at a concentration of 1.66 µg/ml and 60% supercoiling.

### EXPERIMENT 15 : Preparation of Phosphatidylcholine-Containing Microparticles Containing CMVluc DNA

pBKCMVluc plasmid DNA was precipitated in ethanol and resuspended in a solution of TE Ph 8.0/10% sucrose. A lecithin lipid preparation (Lucas Meyer, Catalog No. LECI-PC35F), which is enriched in phosphatidylcholine ("PC"), was added to the DNA solution in varying amounts (vol/vol) as indicated in Tables 7 and 8.

The lipid preparation initially formed a large aggregate after addition to the DNA solution. The aggregate was dispersed into smaller aggregates following vortexing for 20 seconds. After gentle agitation for 30 minutes at room temperature, the PC formed a colloidal suspension.

Lecithin-containing microparticles were formed by adding the suspension to a PLGA/DCM solution and proceeding as described in Experiment 14, above. The observed diameters for the microparticles ranged from 1-10µm.

Tables 7 and 8 provide the concentration of plasmid DNA in the microparticle (expressed in micograms of DNA per mg of polymeric material), the percent supercoiling (SC), and the percentage of starting plasmid DNA encapsulated in microparticles made using DNA resuspended in TE or TE plus 10% sucrose and various concentrations of lecithin. Final concentrations are shown.

**Table 7**

| | **µg/mg** | **%SC** | **% encap** |
|---|---|---|---|
| 10% sucrose, TE Ph 8.0 | 2.79 | 55 | 46.5 |
| 0.3 µl (0.1% lecithin) | 2.78 | 55 | 46.3 |
| 1.5 µl (0.5% lecithin) | 2.55 | 55 | 42.5 |
| 3 µl (1.0% lecithin) | 2.67 | 55 | 44.5 |

**Table 8**

| | **µg/mg** | **%SC** |
|---|---|---|
| TE | 2.39 | 40 |
| 1% lecithin/TE | 2.7 | 40 |
| 5% lecithin/TE | 1.56 | 50 |
| 10% lecithin/TE | 1.23 | 50 |

Table 7 demonstrates that addition of lecithin to an initial concentration of 0-1.0% did not significantly affect properties of the encapsulated DNA, as indicated by the final concentration of DNA in the particle, the percent supercoiling, or the percent of DNA encapsulated.

Table 8 reveals that lecithin present at an initial concentration of 5% or 10% resulted in increased supercoiling and a lower concentration of DNA relative to microparticles prepared using no lecithin or 1% lecithin.

### EXPERIMENT 16 : In vitro Release Properties of Lipid Microparticles

The amount of DNA released from microparticles was determined by preparing microparticles containing DNA and then resuspending the microparticles in an aqueous medium and assaying the supernatant for the presence of DNA using the indicator dye PicoGreen.

Approximately 150 mg of microparticles prepared in TE alone or in TE with CTAB were dissolved in 15 ml TE and injected into a Slide-A-Lyser ™ membrane (M.W. cut off, 10,000), which was then placed in 1 liter of TE at 37°C and stirred. Samples were removed with a syringe at time points, and a 75µl aliquot of was centrifuged at 14k rpm for 5 min. Supernatant was removed and a fraction of this was assayed using PicoGreen.

Fig. 10 shows the percentage of DNA released over time from microparticles prepared using DNA resuspended in TE or CTAB. The percentage of DNA released from TE microparticles increased from slightly less than 20% after 7 days to about 40% after 42 days. In contrast, the percentage of DNA released from CTAB microparticles increased from about 60% after 7 days to over 80% after 42 days. These data demonstrate that CTAB increases the amount of DNA released from microparticles.

Release of DNA from lipid-containing microparticles was also examined in microparticles prepared using TE, TE/10% sucrose, 0.04% lecithin, and 0.04% OVOTHIN 160 lipid. Microparticles containing plasmid DNA were resuspended in TE, and release was assayed by PicoGreen analysis.

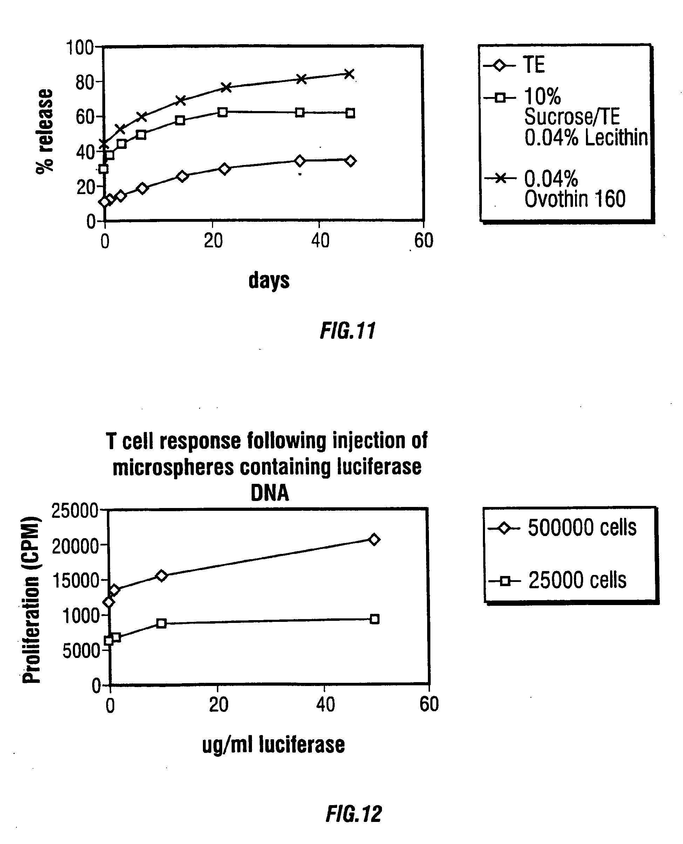

Fig. 11 shows the percentage of DNA released with time from microparticles prepared using the various lipids. The percentage of DNA released from microparticles prepared using 0.04% lecithin or 0.04% OVOTHIN™ 160 was about 80% after 50 days.

In contrast, about 20% of the DNA was released after 50 days from microparticles prepared using TE, and about 60% of DNA was released from microparticles prepared using 10% sucrose/TE. These results demonstrate that the presence of lipid in the microparticles increases the amount of DNA released from the microparticles.

### EXPERIMENT 17 : T cell Proliferation Assays Following Administration of Lipid-Containing Microparticles

Balb/c mice were injected intravenously with 200 µl of microparticles containing the PBKCMVluc plasmid and OVOTHIN™ lipid preparation. Spleens were harvested 11 weeks after injection and analyzed by a T cell proliferation assay.

RBC were lysed and splenocytes washed, counted, and plated in RPMI media containing 10% FCS at 5x10⁵ or 2.5x10⁵ cells/well in 96 well flat bottom plates.

Luciferase antigen (Promega Corp, Madison WI) was added at concentrations ranging from 1 to 50 µg/ml. Studies were conducted using either 250,000 or 500,000 cells per well. The cells were incubated at 37°C for 5 days, after which H³ thymidine was added to each well. 24 hours after addition of H³ thymidine, the cells were harvested on a TOMTEC™ cell harvester and their radioactivity determined.

The results from the studies are shown in Fig. 12. Antigen-proliferative responses were detected using both 250,000 cells and 500,000 cells. These results demonstrate that the injected microparticles elicited a T cell response specific for the encoded luciferase.

## Claims

1. A microparticle for use in a method of therapeutic treatment comprising administering a nucleic acid molecule to an animal by injection into the animal, the microparticle being less than 20 microns in diameter and comprising:
a polymeric matrix;
a phospholipid;
a carbohydrate; and
a nucleic acid molecule, wherein the microparticle is not encapsulated in a liposome and the microparticle does not comprise a cell.

2. The microparticle for use in accordance with claim 1, wherein the nucleic acid molecule is circular.

3. The microparticle for use in accordance with claim 1 or 2, wherein the nucleic acid molecule is a plasmid.

4. The microparticle for use in accordance with any one of the preceding claims, wherein the nucleic acid molecule comprises an expression control sequence operatively linked to a coding sequence.

5. The microparticle for use in accordance with any one of the preceding claims, further comprising a targeting molecule.

6. The microparticle for use in accordance with claim 1, wherein the nucleic acid molecule comprises an expression control sequence operatively linked to a coding sequence, wherein the coding sequence encodes an expression product selected from the group consisting of:
(a) a polypeptide at least 7 amino acids in length, having a sequence essentially identical to the sequence of (i) a fragment of a naturally-occurring mammalian protein; or (ii) a fragment of a naturally-occurring protein from an infectious agent which infects a mammal; or (iii) a plurality of the fragments of (i), linked in tandem; or (iv) a plurality of the fragments of (ii), linked in tandem;
(b) a peptide having a length and sequence which permit it to bind to an MHC class I or II molecule;
(c) a polypeptide consisting of at least two peptides of (b) either linked in tandem or sharing an overlapping sequence; and
(d) any of (a), (b), or (c) linked to a trafficking sequence,
provided that the expression product optionally includes an amino terminal methionine residue, and further provided that the expression product does not have an amino acid sequence identical to that of a full-length, naturally-occurring protein.

7. The microparticle for use in accordance with claim 6, wherein the expression product is a polypeptide consisting of at least two peptides of (b) linked in tandem, wherein the at least two peptides of (b) are not identical.

8. The microparticle for use in accordance with claim 6, wherein the expression product is a polypeptide consisting of at least two overlapping peptides of (b).

9. The microparticle for use in accordance with claim 6, wherein the expression product comprises a peptide having a length and sequence which permit it to bind an MHC class I or class II molecule.

10. The microparticle for use in accordance with any of claims 6-9, wherein the expression product is immunogenic.

11. The microparticle for use in accordance with any of claims 6-10, wherein the expression product (1) has an amino acid sequence that differs by no more than 25% from the sequence of a naturally occurring peptide recognized by a T cell; and (2) is recognized by the T cell.

12. The microparticle for use in accordance with any of claims 6-11, wherein the expression product consists of an amino acid sequence at least 50% identical to the sequence of a fragment at least 10 amino acids in length of a protein selected from the group consisting of myelin basic protein (MBP), proteolipid protein (PLP), invariant chain, GAD65, islet cell antigen, desmoglein, α-crystatlin, and β-crystallin, wherein the fragment binds to an MHC class II molecule.

13. The microparticle for use in accordance with any of claims 6-12, wherein the expression product comprises an amino acid sequence essentially identical to a sequence selected from the group consisting of SEQ ID NOS 1-46 and an antigenic portion of a tumor antigen.

14. The microparticle for use in accordance with any of claims 6-13, wherein the expression product comprises a trafficking sequence selected from the group consisting of a sequence which trafficks to endoplasmic reticulum, a sequence which trafficks to a lysosome, a sequence which trafficks to an endosome, a sequence which trafficks to an intracellular vesicle, and a sequence which trafficks to the nucleus.

15. The microparticle for use in accordance with claim 13, wherein the tumor antigen is selected from the group consisting of the proteins listed in Table 3.

16. The microparticle for use in accordance with claim 6, wherein the expression product comprises an amino acid sequence essentially identical to the sequence of an antigenic fragment of a protein naturally expressed by an infectious agent selected from the group consisting of a virus, a bacterium, and a parasitic eukaryote.

17. The microparticle for use in accordance with claim 16, wherein the infectious agent is selected from the group consisting of herpes simplex virus, hepatitis B virus, hepatitis C virus, *Plasmodium* species, *Chlamydia,* mycobacteria, human papilloma virus, and human immunodeficiency virus.

18. The microparticle for use in accordance with any of claims 1-17, wherein the phospholipid is a phosphatidylethanolamine.

19. The microparticle for use in accordance with any of claims 1-17, wherein the phospholipid is a phosphatidylcholine, a phosphatidylserine, or a phosphatidylinositol.

20. The microparticle for use in accordance with any one of the preceding claims, wherein the carbohydrate is sucrose.

21. The microparticle for use in accordance with any one of the preceding claims, further comprising a second lipid.

22. The microparticle for use in accordance with any one of the preceding claims, wherein the polymeric matrix is biodegradable.

23. The microparticle for use in accordance with any one of the preceding claims, wherein the polymeric matrix comprises a synthetic, biodegradable copolymer, preferably poly-lactic-*co*-glycolic acid (PLGA).

24. The microparticle for use in accordance with claim 23, wherein the ratio of lactic acid to glycolic acid in the copolymer is within the range of 1:2 to 4:1 by weight, preferably 65:35 by weight.

25. A preparation of microparticles for use in a method of therapeutic treatment comprising administering a nucleic acid molecule to an animal by injection into the animal, the preparation of microparticles comprising a plurality of the microparticles of any one of the preceding claims.

26. The preparation for use in accordance with claim 25, wherein at least 50% of the nucleic acid molecules in the microparticles are supercoiled.

27. The preparation for use in accordance with claim 25 or 26, wherein at least 90% of the microparticles have a diameter less than about 11 microns.

28. The preparation for use in accordance with any one of claims 25 to 27, wherein the microparticles are suspended in a pharmaceutically acceptable carrier.

## Patentansprüche

1. Mikropartikel zur Verwendung bei einer Methode der therapeutischen Behandlung, umfassend das Verabreichen eines Nucleinsäuremoleküls einem Tier durch Injektion in das Tier, wobei das Mikropartikel im Durchmesser kleiner als 20 Mikron ist und Folgendes umfasst:
eine Polymermatrix;
ein Phospholipid;
ein Kohlehydrat; und
ein Nucleinsäuremolekül, wobei das Mikropartikel nicht in einem Liposom verkapselt ist und das Mikropartikel keine Zelle umfasst.

2. Mikropartikel zur Verwendung nach Anspruch 1, wobei das Nucleinsäuremolekül rund ist.

3. Mikropartikel zur Verwendung nach Anspruch 1 oder 2, wobei das Nucleinsäuremolekül ein Plasmid ist.

4. Mikropartikel zur Verwendung nach einem der vorhergehenden Ansprüche, wobei das Nucleinsäuremolekül eine Expressionskontrollsequenz umfasst, die operativ an eine Kodiersequenz geknüpft ist.

5. Mikropartikel zur Verwendung nach einem der vorhergehenden Ansprüche, des Weiteren ein targetierendes Molekül umfassend.

6. Mikropartikel zur Verwendung nach Anspruch 1, wobei das Nucleinsäuremolekül eine Expressionskontrollsequenz umfasst, die operativ an eine Kodiersequenz geknüpft ist, wobei die Kodiersequenz ein Expressionsprodukt ausgewählt aus der Gruppe kodiert bestehend aus:
(a) einem Polypeptid, das eine Länge von mindestens 7 Aminosäuren und eine Sequenz aufweist, die im Wesentlichen identisch ist mit der Sequenz von (i) einem Fragment eines natürlich vorkommenden Säugerproteins; oder (ii) einem Fragment eines natürlich vorkommenden Proteins aus einem infektiösen Agens, das einen Säuger infiziert; oder (iii) mehreren der Fragmente von (i), die hintereinander mit einander verknüpft sind; oder (iv) mehreren der Fragmente von (ii), die hintereinander mit einander verknüpft sind;
(b) einem Peptid, das eine Länge und Sequenz aufweist, die es ihm erlauben, sich an ein MHC-Klasse I- oder -II-Molekül zu binden;
(c) einem Polypeptid, das aus mindestens zwei Peptiden von (b) besteht, die entweder hintereinander miteinander verknüpft sind oder an einer überlappenden Sequenz teilhaben; und
(d) irgendwelchen von (a), (b) oder (c), die an eine Trafficking-Sequenz angeknüpft sind,
vorausgesetzt, dass das Expressionsprodukt wahlweise einen aminoterminalen Methioninrest umfasst und des Weiteren, vorausgesetzt, dass das Expressionsprodukt keine Aminosäuresequenz aufweist, die mit derjenigen eines natürlich vorkommenden Volllängen-Proteins identisch ist.

7. Mikropartikel zur Verwendung nach Anspruch 6, wobei das Expressionsprodukt ein Polypeptid ist, das aus mindestens zwei Peptiden von (b) besteht, die hintereinander miteinander verknüpft sind, wobei mindestens zwei Peptide von (b) nicht identisch sind.

8. Mikropartikel zur Verwendung nach Anspruch 6, wobei das Expressionsprodukt ein Polypeptid ist, das aus mindestens zwei überlappenden Peptiden von (b) besteht.

9. Mikropartikel zur Verwendung nach Anspruch 6, wobei das Expressionsprodukt ein Peptid umfasst, das eine Länge und Sequenz aufweist, die es ihm erlauben, sich an ein MHC-Klasse I- oder -Klasse II-Molekül zu binden.

10. Mikropartikel zur Verwendung nach einem der Ansprüche 6-9, wobei das Expressionsprodukt immunogen ist.

11. Mikropartikel zur Verwendung nach einem der Ansprüche 6-10, wobei das Expressionsprodukt (1) eine Aminosäuresequenz aufweist, die sich um nicht mehr als 25 % von der Sequenz eines natürlich vorkommenden Peptids unterscheidet, das von einer T-Zelle erkannt wird; und (2) von der T-Zelle erkannt wird.

12. Mikropartikel zur Verwendung nach einem der Ansprüche 6-11, wobei das Expressionsprodukt aus einer Aminosäuresequenz besteht, die mit der Sequenz eines Fragments einer Länge von mindestens 10 Aminosäuren eines Proteins mindestens 50 % identisch ist, das aus der Gruppe ausgewählt ist bestehend aus myelin-basischem Protein (MBP), Proteolipidprotein (PLP), invarianter Kette, GAD65, Langerhans'sche Inseln-Antigen, Desmoglein, α-kristallin, und ß-kristallin, wobei das Fragment sich an ein MHC-Klasse II-Molekül bindet.

13. Mikropartikel zur Verwendung nach einem der Ansprüche 6-12, wobei das Expressionsprodukt eine Aminosäuresequenz umfasst, die im Wesentlichen mit einer Sequenz identisch ist ausgewählt aus der Gruppe bestehend aus SEQ ID NO: 1-46 und einem antigenen Anteil eines Tumorantigens.

14. Mikropartikel zur Verwendung nach einem der Ansprüche 6-13, wobei das Expressionsprodukt eine Trafficking-Sequenz umfasst ausgewählt aus der Gruppe bestehend aus einer Sequenz, die sich zu endoplasmatischem Retikulum bewegt, einer Sequenz, die sich zu einem Lyosom bewegt, einer Sequenz, die sich zu einem Endosom bewegt, einer Sequenz, die sich zu einem intrazellulären Gefäß bewegt und einer Sequenz, die sich zu einem Nukleus bewegt.

15. Mikropartikel zur Verwendung nach Anspruch 13, wobei das Tumorantigen aus der Gruppe ausgewählt ist bestehend aus den in Tabelle 3 aufgelisteten Proteinen.

16. Mikropartikel zur Verwendung nach Anspruch 6, wobei das Expressionsprodukt eine Aminosäuresequenz umfasst, die im Wesentlichen mit der Sequenz eines antigenen Fragments eines Proteins identisch ist, das natürlich durch ein infektiöses Agens ausgewählt aus der Gruppe bestehend aus einem Virus, einem Bakterium und einem parasitischen Eukaryoten exprimiert wird.

17. Mikropartikel zur Verwendung nach Anspruch 16, wobei das infektiöse Agens aus der Gruppe ausgewählt ist bestehend aus dem Herpes simplex-Virus, dem Hepatitis B-Virus, dem Hepatitis C-Virus, der Plasmodium-Spezies, Chlamydia, Mykobakterien, dem menschlichen Papilloma-Virus und dem menschlichen Immunmangel-Virus.

18. Mikropartikel zur Verwendung nach einem der Ansprüche 1-17, wobei das Phospholipid ein Phosphatidylethanolamin ist.

19. Mikropartikel zur Verwendung nach einem der Ansprüche 1-17, wobei das Phospholipid ein Phosphatidylcholin, ein Phosphatidylserin oder ein Phosphatidylinositol ist.

20. Mikropartikel zur Verwendung nach einem der vorhergehenden Ansprüche, wobei das Kohlehydrat Saccharose ist.

21. Mikropartikel zur Verwendung nach einem der vorhergehenden Ansprüche, des Weiteren ein zweites Lipid umfassend.

22. Mikropartikel zur Verwendung nach einem der vorhergehenden Ansprüche, wobei die polymere Matrix biologisch abbaubar ist.

23. Mikropartikel zur Verwendung nach einem der vorhergehenden Ansprüche, wobei die polymere Matrix ein synthetisches, biologisch abbaubares Copolymer, bevorzugt Poly-milch-co-glycolsäure (PLGA) umfasst.

24. Mikropartikel zur Verwendung nach Anspruch 23, wobei das Verhältnis von Milchsäure zu Glykolsäure in dem Copolymer im Bereich von 1:2 bis 4:1, auf das Gewicht bezogen, bevorzugt 65:35, auf das Gewicht bezogen, liegt.

25. Zubereitung von Mikropartikeln zur Verwendung bei einer Methode der therapeutischen Behandlung umfassend das Verabreichen eines Nucleinsäuremoleküls einem Tier durch Injektion in das Tier, wobei die Zubereitung von Mikropartikeln mehrere der Mikropartikel nach einem der vorhergehenden Ansprüche umfasst.

26. Zubereitung zur Verwendung nach Anspruch 25, wobei mindestens 50 % der Nucleinsäuremoleküle in den Mikropartikeln supercoiled sind.

27. Zubereitung zur Verwendung nach Anspruch 25 oder 26, wobei mindestens 90 % der Mikropartikel einen Durchmesser von weniger als etwa 11 Mikron aufweisen.

28. Zubereitung zur Verwendung nach einem der Ansprüche 25 bis 27, wobei die Mikropartikel in einem pharmazeutisch akzeptablen Träger suspendiert sind.

## Revendications

1. Microparticule pour l'utilisation dans un procédé de traitement thérapeutique comprenant l'administration d'une molécule d'acide nucléique à un animal par injection à l'animal, la microparticule étant inférieure à 20 microns de diamètre et comprenant:
une matrice polymère;
un phospholipide;
un hydrate de carbone; et
une molécule d'acide nucléique, dans laquelle la microparticule n'est pas encapsulée dans un liposome et la microparticule ne comprend pas de cellule.

2. Microparticule pour l'utilisation selon la revendication 1, dans laquelle la molécule d'acide nucléique est circulaire.

3. Microparticule pour l'utilisation selon la revendication 1 ou 2, dans laquelle la molécule d'acide nucléique est un plasmide.

4. Microparticule pour l'utilisation selon l'une quelconque des revendications précédentes, dans laquelle la molécule d'acide nucléique comprend une séquence de contrôle d'expression fonctionnellement liée à une séquence codante.

5. Microparticule pour l'utilisation selon l'une quelconque des revendications précédentes, comprenant en outre une molécule ciblante.

6. Microparticule pour l'utilisation selon la revendication 1, dans laquelle la molécule d'acide nucléique comprend une séquence de contrôle d'expression fonctionnellement liée à une séquence codante, dans laquelle la séquence codante code un produit d'expression choisi parmi le groupe constitué de:
(a) un polypeptide d'au moins 7 acides aminés de longueur, ayant une séquence essentiellement identique à la séquence de (i) un fragment d'une protéine de mammifère d'origine naturelle; ou (ii) un fragment d'une protéine d'origine naturelle provenant d'un agent infectieux qui infecte un mammifère; ou (iii) une pluralité de fragments de (i), liés en tandem; ou (iv) une pluralité de fragments de (ii), liés en tandem;
(b) un peptide ayant une longueur et une séquence qui lui permettent de se lier à une molécule du CMH de classe I ou II;
(c) un polypeptide constitué d'au moins deux peptides de (b) soit liés en tandem soit partageant une séquence chevauchante; et
(d) n'importe lequel parmi (a), (b) ou (c) lié à une séquence de "trafficking",
à condition que le produit d'expression inclue optionnellement un résidu méthionine amino terminal, et en outre à condition que le produit d'expression n'ait pas de séquence d'acides aminés identique à celle d'une protéine d'origine naturelle de pleine longueur.

7. Microparticule pour l'utilisation selon la revendication 6, dans laquelle le produit d'expression est un polypeptide constitué d'au moins deux peptides de (b) liés en tandem, dans laquelle les au moins deux peptides de (b) ne sont pas identiques.

8. Microparticule pour l'utilisation selon la revendication 6, dans laquelle le produit d'expression est un polypeptide constitué d'au moins deux peptides chevauchants de (b).

9. Microparticule pour l'utilisation selon la revendication 6, dans laquelle le produit d'expression comprend un peptide ayant une longueur et une séquence qui lui permettent de se lier à une molécule du CMH de classe I ou de classe II.

10. Microparticule pour l'utilisation selon l'une quelconque des revendications 6 à 9, dans laquelle le produit d'expression est immunogène.

11. Microparticule pour l'utilisation selon l'une quelconque des revendications 6 à 10, dans laquelle le produit d'expression (1) a une séquence d'acides aminés qui diffère de pas plus de 25 % de la séquence d'un peptide d'origine naturelle reconnu par une cellule T; et (2) est reconnu par la cellule T.

12. Microparticule pour l'utilisation selon l'une quelconque des revendications 6 à 11, dans laquelle le produit d'expression est constitué d'une séquence d'acides aminés au moins à 50 % identique à la séquence d'un fragment d'au moins 10 acides aminés de longueur d'une protéine choisie parmi le groupe constitué de la protéine de base de la myéline (PBM), de la protéine protéolipidique (PLP), de la chaîne invariante, GAD65, d'un antigène de cellule d'îlots pancréatiques, de la desmogléine, de l'α-cristalline, et de la β-cristalline, dans laquelle le fragment se lie à une molécule du CMH de classe II.

13. Microparticule pour l'utilisation selon l'une quelconque des revendications 6 à 12, dans laquelle le produit d'expression comprend une séquence d'acides aminés essentiellement identique à une séquence choisie parmi le groupe constitué des SEQ ID NOS: 1 à 46 et d'une portion antigénique d'un antigène tumoral.

14. Microparticule pour l'utilisation selon l'une quelconque des revendications 6 à 13, dans laquelle le produit d'expression comprend une séquence de "trafficking" choisie parmi le groupe constitué d'une séquence qui effectue le trafic vers le réticulum endoplasmique, une séquence qui effectue le trafic vers un lysosome, une séquence qui effectue le trafic vers un endosome, une séquence qui effectue le trafic vers une vésicule intracellulaire et une séquence qui effectue le trafic vers le noyau.

15. Microparticule pour l'utilisation selon la revendication 13, dans laquelle l'antigène tumoral est choisi parmi le groupe constitué des protéines listées dans le tableau 3.

16. Microparticule pour l'utilisation selon la revendication 6, dans laquelle le produit d'expression comprend une séquence d'acides aminés essentiellement identique à la séquence d'un fragment antigénique d'une protéine naturellement exprimée par un agent infectieux choisi parmi le groupe constitué d'un virus, d'une bactérie et d'un eucaryote parasite.

17. Microparticule pour l'utilisation selon la revendication 16, dans laquelle l'agent infectieux est choisi parmi le groupe constitué du virus de l'herpès simplex, du virus de l'hépatite B, du virus de l'hépatite C, des espèces du genre *Plasmodium*, *Chlamydia*, des mycobactéries, du virus du papillome humain et du virus de l'immunodéficience humaine.

18. Microparticule pour l'utilisation selon l'une quelconque des revendications 1 à 17, dans laquelle le phospholipide est une phosphatidyléthanolamine.

19. Microparticule pour l'utilisation selon l'une quelconque des revendications 1 à 17, dans laquelle le phospholipide est une phosphatidylcholine, une phosphatidylsérine ou un phosphatidylinositol.

20. Microparticule pour l'utilisation selon l'une quelconque des revendications précédentes, dans laquelle l'hydrate de carbone est le saccharose.

21. Microparticule pour l'utilisation selon l'une quelconque des revendications précédentes, comprenant en outre un second lipide.

22. Microparticule pour l'utilisation selon l'une quelconque des revendications précédentes, dans laquelle la matrice polymère est biodégradable.

23. Microparticule pour l'utilisation selon l'une quelconque des revendications précédentes, dans laquelle la matrice polymère comprend un copolymère de synthèse, biodégradable, de préférence de l'acide poly-lactique-co-glycolique (PLGA).

24. Microparticule pour l'utilisation selon la revendication 23, dans laquelle le rapport de l'acide lactique à l'acide glycolique dans le copolymère se situe dans la plage de 1:2 à 4:1 en poids, de préférence 65:35 en poids.

25. Préparation de microparticules pour l'utilisation dans un procédé de traitement thérapeutique comprenant l'administration d'une molécule d'acide nucléique à un animal par l'injection à l'animal, la préparation de microparticules comprenant une pluralité de microparticules selon l'une quelconque des revendications précédentes.

26. Préparation pour l'utilisation selon la revendication 25, dans laquelle au moins 50 % des molécules d'acide nucléique dans les microparticules sont surenroulées.

27. Préparation pour l'utilisation selon la revendication 25 ou 26, dans laquelle au moins 90 % des microparticules ont un diamètre inférieur à environ 11 microns.

28. Préparation pour l'utilisation selon l'une quelconque des revendications 25 à 27, dans laquelle les microparticules sont placées en suspension dans un support pharmaceutiquement acceptable.
